# EUROPEAN PATENT SPECIFICATION

(11) **EP 4 148 117 B1**
(45) Date of publication and mention of the grant of the patent: **31.12.2025**
(21) Application number: 22178933.2
(22) Date of filing: 14.06.2022
(51) Int. Cl.: C12M 1/32, C12M 1/00, C12M 1/12, C12M 3/06

(54) **CELL CULTURE APPARATUS, METHODS FOR CELL CULTIVATION BY USING THE SAME, CELL CULTURE INCUBATOR COMPRISING THE SAME**
ZELLKULTIVIERUNGSVORRICHTUNG, VERFAHREN ZUR ZELLKULTIVIERUNG UNTER VERWENDUNG DERSELBEN, ZELLKULTIVIERUNGSINKUBATOR DAMIT
APPAREIL DE CULTURE CELLULAIRE, PROCÉDÉS DE CULTURE CELLULAIRE L'UTILISANT, INCUBATEUR DE CULTURE CELLULAIRE LE COMPRENANT

(30) Priority: 10.09.2021 EP 21195867
(43) Date of publication of application: 15.03.2023
(73) Proprietor: Finnadvance Oy, 90220 Oulu (FI)
(72) Inventor: Singh, Prateek, 90220 Oulu (FI); Nguyen, Hoang Tuan, 90220 Oulu (FI)
(74) Representative: Papula Oy

(56) References cited:
- WO-A1-2020/081740
- WO-A1-2020/109421
- AU-B2- 2018 283 184
- US-A1- 2013 059 322

## Description

### TECHNICAL FIELD

The present disclosure relates generally to the field of biotechnology. In particular, the present disclosure relates to a cell culture apparatus, a method for affecting a cell culture by using the cell culture apparatus, uses of the cell culture apparatus, and a cell culture incubator comprising the cell culture apparatus.

### BACKGROUND

Cell culture or cell cultivation involves growing cells of desired type(s) outside a living body under controlled *in vitro* conditions to sustain cell growth and viability. The cell culture is widely used in different biotechnology branches, including cell biology, tissue engineering, biomedical engineering, cell differentiation studies, cell-based biosensors, cell-cell interaction, cell-signaling, cell-migration, physiological and pathophysiological studies, etc.

Environmental conditions created for cultured cells should resemble as closely as possible the conditions experienced by the same cells *in vivo.* This may be done by performing the cell culture in large vessels, such as dishes, spinner and shaker flasks, etc. However, the cell culture conditions provided by these vessels do not truly represent the *in vivo* environment of the cells being cultured. Moreover, since these vessels have a large volume, they consume significant amounts of reagents, culture media, chemicals, etc., thereby making it difficult to control and/or alter the cell culture conditions.

With the advent of microfluidics, new devices and methods configured to cultivate various types of cells, like adherent and non-adherent, under uniform and controlled *in vitro* conditions have been developed. Unlike the conventional cell culture methods based on the above-mentioned vessels, microfluidic cell culture methods may provide continuous nutrient (culture fluid or medium) supply, waste removal, flexibility of schedules, and high automation capability. The less consumption of fluids, their low volumes and therefore the reduced time and cost of cell cultivation make these microfluidic methods particularly interesting for cell-based assays. The main goals of microfluidic cell culture devices are to mimic closely *in vivo* cellular microenvironments and to maintain simplicity for reproducible results.

A silicone insert with two wells made of silicone, wherein the wells having a defined cell-free gap has been previously developed. These inserts are reported as suitable for studying wound healing, using in migration assays, 2D invasion assays, and co-cultivation of cells. These inserts have a defined 500 µm cell free gap between the two wells, are disclosed as having no leakage during cultivation, and leaving no material left behind after the insert's removal. The insert is prepared on the surface of a Petri dish, then cells are seeded in the wells of the insert, and after waiting for cells attachment to the surface of the Petri dish, the insert is removed. A cell-free gap is created and leaving the attached cells on the surface of the Petri dish. Medium is added on top of the attached cells and cell migration and wound healing may be studied. However, these inserts require, e.g., a Petri dish or a flat, clean surface, for these applications.

Another technique of studying migration of cells is to use a well having a central cell-free detection zone created using a water-soluble bio-compatible gel. Cells are added to the well, the well is incubated for cell attachment, upon attachment of the cells the water-soluble bio-compatible gel dissolves within 60 minutes, allowing the cells to migrate into the detection zone at the center of each well. However, the use of water-soluble bio-compatible gel may be toxic for some cells and adds unnatural compounds to the cell culture.

Still another technique to study cell migration is to use a stopper barrier that create a central cell-free detection zone. The stopper is added on the surface of the well, cells are added around the stopper to the well, the cells are incubated for cell attachment to the surface of the well, the stopper is removed, and cell migration towards the detection zone may be studied, e.g., from below the well. An example from the prior art is WO 2020/081740 A1 which discloses an apparatus with a central volume of flanked by two lateral volumes in direct fluid contact at their bottom.

However, the limiting factors of the current techniques are their low throughput and incompatibility with available liquid handling systems and imaging systems due to the discrete arrangement. Furthermore, fluid flow control in the current technology is not possible and the current technology is not flexible enough to adapt to different resource settings and different cell-based assays. Furthermore, the current technology does not enable one to remove cells from a cell culture in a microfluidic cell culture device or apparatus in a controlled manner and thus, to study wound healing using a microfluidic cell culture device or apparatus. Current microfluidic platforms suffer from incompatibility with imaging systems due to obstructing microfluidic channels.

### SUMMARY

This summary is provided to introduce a selection of concepts in a simplified form that are further described below in the detailed description. This summary is not intended to identify key features of the present disclosure, nor is it intended to be used to limit the scope of the present disclosure.

It is an objective of the present disclosure to provide a technical solution that enables high-throughput microfluidic cell cultivation. More specifically, an objective of the present disclosure to provide a technical solution that enables studying wound healing and/or cell, spheroid, and/or organoid formation.

The objective above is achieved by the features of the independent claims in the appended claims. Further embodiments and examples are apparent from the dependent claims, the detailed description, and the accompanying drawings.

According to a first aspect, a cell culture apparatus is provided, the cell culture apparatus comprising:
one or more cell culture modules, each cell culture module of the one or more cell culture modules comprising:
   - at least two culture medium reservoirs each having a top part and a bottom part, the top part having an inlet for a culture medium and the bottom part having an outlet for the culture medium;
   - a first chamber arranged between the at least two culture medium reservoirs such that the first chamber and the at least two culture medium reservoirs are aligned with each other in a first direction;
   - a second chamber arranged under the first chamber and aligned with the first chamber in a second direction, the second direction having an angle being more than 0 degrees but 90 degrees or less in respect to the first direction, the second chamber having a bottom part having at least two lateral holes;
   - a wall made of a wall material, the wall being arranged between the first chamber and the second chamber; and
   - at least two flow channels, each of the at least two flow channels being configured to pass a cell culture medium therethrough, and each of the at least two flow channels connecting the outlet of the bottom part of one of the at least two culture medium reservoirs to one of the at least two lateral holes of the bottom part of the second chamber,
wherein the bottom wall of the second chamber is made of an elastic bottom wall material configured to deform in a third direction, the third direction being opposite to the second direction, in response to a force applied on the bottom side of the bottom wall of the second chamber and/or the wall material of the wall is an elastic wall material configured to deform in the second direction in response to a force applied on the first chamber side of the wall.

According to a second aspect, a cell culture incubator is provided, the cell culture incubator comprising:
a cell culture apparatus as disclosed in the present disclosure; and
a pipetting station configured to feed the fluid or culture medium to each of the at least two culture medium reservoirs in each cell culture module of the one or more cell culture modules.

According to a third aspect, a method for affecting a cell culture is provided, the method comprising:
(a) providing a cell culture apparatus as disclosed in the present disclosure, wherein each second chamber of each cell culture module of the one or more cell culture modules comprises a cell culture comprising a plurality of cells of a first type of cells;
(b) applying one or more first force
   i) on one or more first locations on the bottom side of the bottom wall of the second chamber such that the bottom wall of the second chamber deforms in the third direction; and/or
   ii) on one or more second locations on the first chamber side of the wall such that the wall deforms in the second direction,
thereby removing at least one cell of from the plurality of cells of the first type of cells of the cell culture and forming a first affected cell culture.

According to a fourth aspect is provided a use of a cell culture apparatus as disclosed in the present disclosure for analyzing cell cultures, in methods for cell cultivation, and for affecting cell cultures.

Other features and advantages of the present disclosure will be apparent upon reading the following detailed description and reviewing the accompanying drawings.

### BRIEF DESCRIPTION OF THE DRAWINGS

The present disclosure is explained below with reference to the accompanying drawings in which:
FIG. 1 shows a block diagram of a cell culture apparatus in accordance with an exemplary embodiment;
FIGs. 2A-2C show different schematic views of a cell culture module included in the apparatus shown in FIG. 1 in accordance with exemplary embodiments, namely: FIG. 2A shows a schematic perspective view of the cell culture module, FIG. 2B shows a schematic side view of the cell culture module, and FIG. 2C shows a schematic top view of the cell culture module;
FIG. 3 shows a sectional view of one of two flow channels of a cell culture module, as taken within the area delimited by oval A in FIG. 2B, in accordance with exemplary embodiments;
FIG. 4 shows a schematic exploded view of a layered structure that represents one possible implementation example of the cell culture module shown in FIGs. 2A-2C;
FIGs. 5A-5C show different schematic views of the cell culture module included in the apparatus shown in FIG. 1 in accordance with an exemplary embodiment, namely: FIG. 5A shows a schematic perspective view of the cell culture module, FIG. 5B shows a schematic side view of the cell culture module, and FIG. 5C shows a schematic top view of the cell culture module;

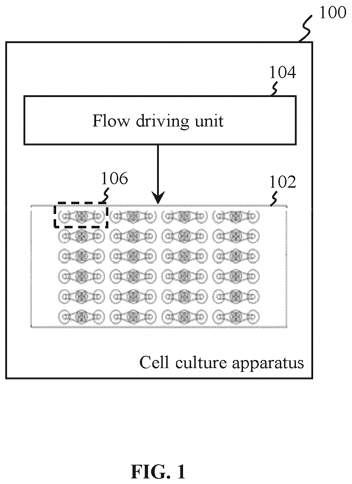

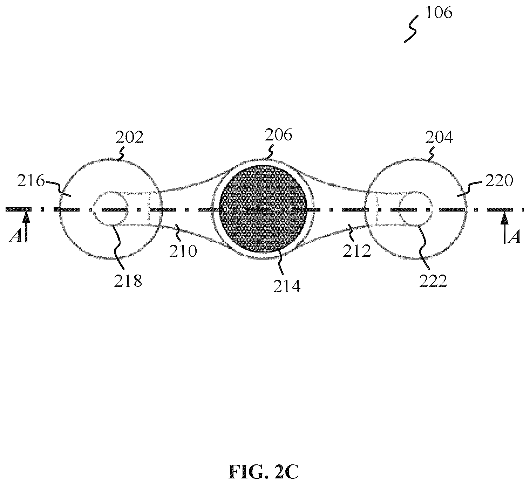

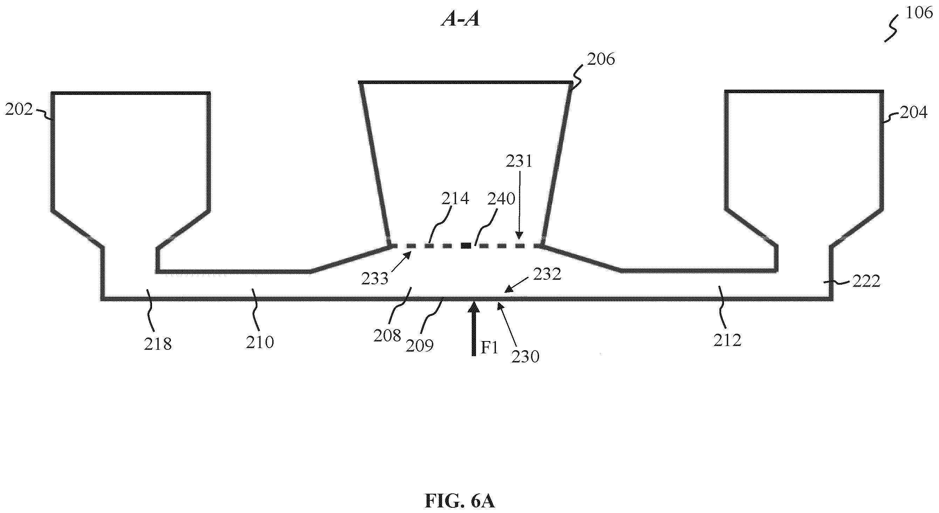

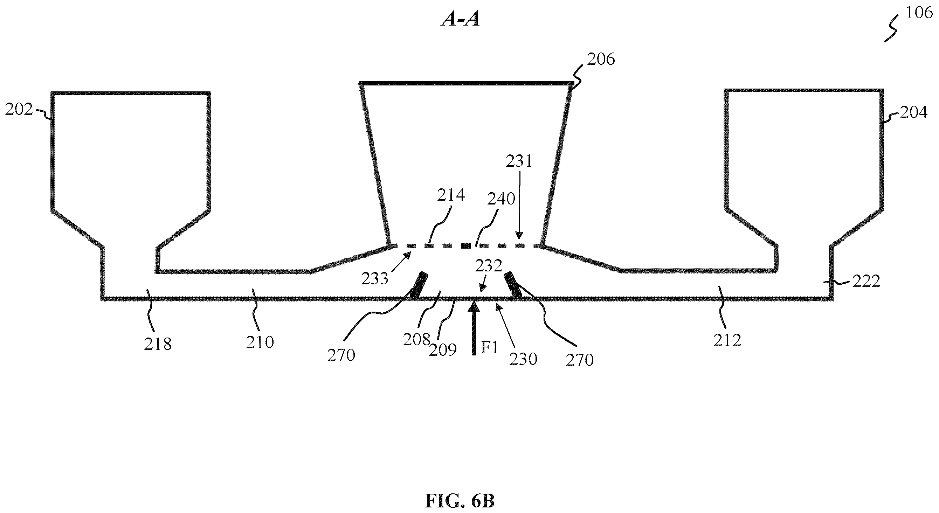

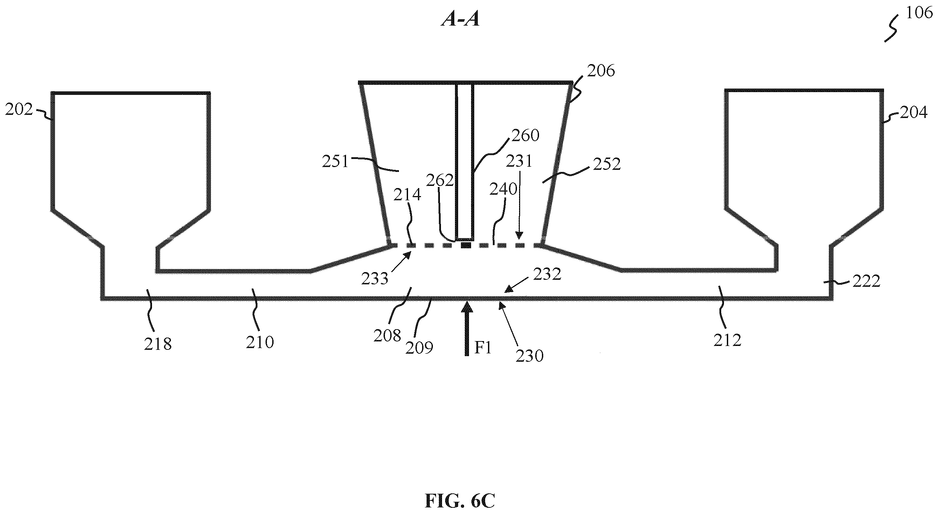

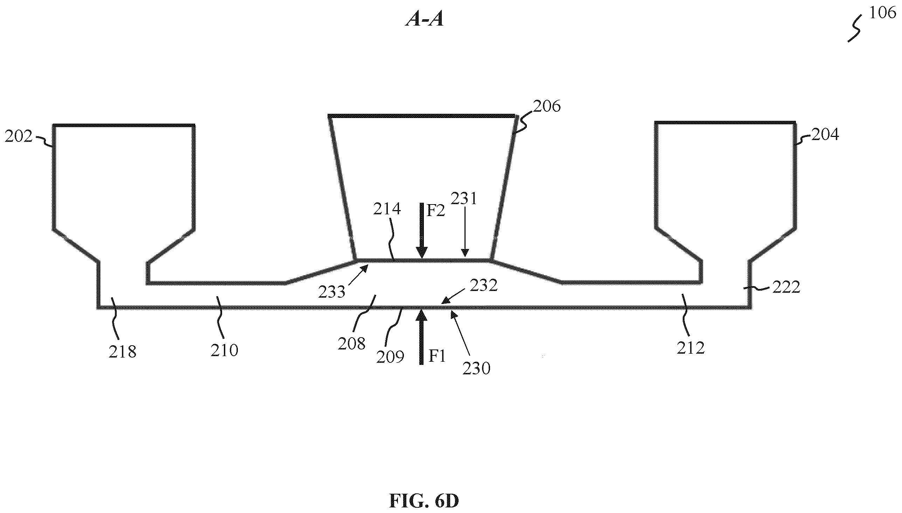

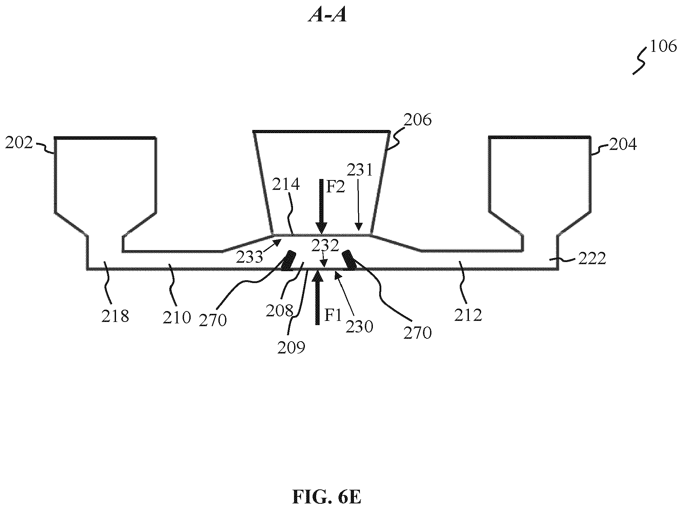

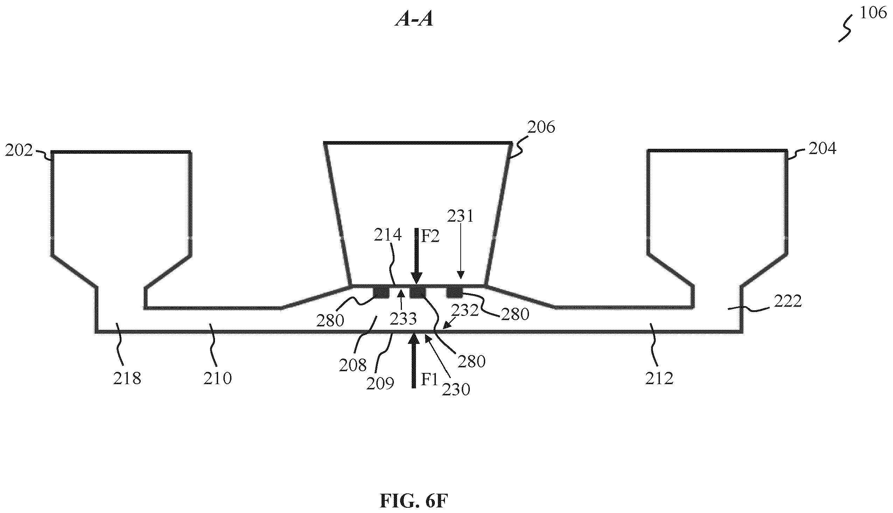

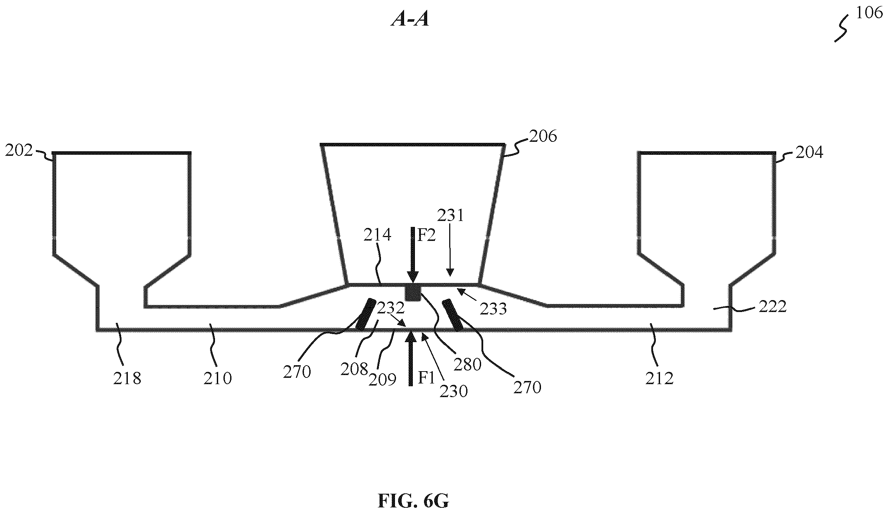

FIGs. 6A-6G show schematic sectional side views of exemplary cell culture modules (as taken along line A-A in FIG. 2C) included in the cell culture apparatus shown in FIG. 1, namely: FIG. 6A shows a schematic side view of the cell culture module (as taken along line A-A in FIG. 2C) in accordance with exemplary embodiments, FIG. 6B shows a schematic sectional side view of the cell culture module (as taken along line A-A in FIG. 2C) in accordance with exemplary embodiments, FIG. 6C shows a schematic sectional side view of the cell culture module (as taken along line A-A in FIG. 2C) in accordance with exemplary embodiments, FIG. 6D shows a schematic sectional side view of the cell culture module (as taken along line A-A in FIG. 2C) in accordance with exemplary embodiments, FIG. 6E shows a schematic sectional side view of the cell culture module (as taken along line A-A in FIG. 2C) in accordance with exemplary embodiments, FIG. 6F shows a schematic sectional side view of the cell culture module (as taken along line A-A in FIG. 2C) in accordance with exemplary embodiments, and FIG. 6G shows a schematic sectional side view of the cell culture module (as taken along line A-A in FIG. 2C) in accordance with exemplary embodiments;
FIG. 7 shows a block diagram of a cell culture incubator in accordance with exemplary embodiments;
FIG. 8 shows a flowchart of a method for affecting a cell culture in accordance with exemplary embodiments; and
FIGs. 9A and 9B show confocal images of a BBB model by using the apparatus shown in FIG. 1.

### DETAILED DESCRIPTION

Various embodiments of the present disclosure are further described in more detail with reference to the accompanying drawings. However, the present disclosure may be embodied in many other forms and should not be construed as limited to any certain structure or function discussed in the following description. In contrast, these embodiments are provided to make the description of the present disclosure detailed and complete.

According to the detailed description, it will be apparent to the ones skilled in the art that the scope of the present disclosure encompasses any embodiment thereof, which is disclosed herein, irrespective of whether this embodiment is implemented independently or in concert with any other embodiment of the present disclosure. For example, apparatuses and/or methods disclosed herein may be implemented in practice using any numbers of the embodiments provided herein. Furthermore, it should be understood that any embodiment of the present disclosure may be implemented using one or more of the elements presented in the appended claims.

The word "exemplary" is used herein in the meaning of "used as an illustration". Unless otherwise stated, any embodiment described herein as "exemplary" should not be construed as preferable or having an advantage over other embodiments.

Any positioning terminology, such as "left", "right", "top", "bottom", "above", "under", "apical", "basal", etc., may be used herein for convenience to describe one element's or feature's relationship to one or more other elements or features in accordance with the figures. It should be apparent that the positioning terminology is intended to encompass different orientations of the structure and device disclosed herein, in addition to the orientation(s) depicted in the figures. As an example, if one imaginatively rotates the structure or device in the figures 90 degrees clockwise, elements or features described as "top" and "bottom" relative to other elements or features would then be oriented, respectively, "right" and "left" relative to the other elements or features. Therefore, the positioning terminology used herein should not be construed as any limitation of the present disclosure.

Furthermore, although the numerative terminology, such as "first", "second", etc., may be used herein to describe various embodiments, elements or features, it should be understood that these embodiments, elements or features should not be limited by this numerative terminology. This numerative terminology is used herein only to distinguish one embodiment, element or feature from another embodiment, element or feature. For example, a first chamber discussed below could be called a second chamber, and vice versa, without departing from the teachings of the present disclosure.

As disclosed herein, a cell may refer to a biological cell, such as a plant cell, an animal cell (e.g., a mammalian cell), a bacterial cell, a fungal cell, or the like. A mammalian cell may be, for example, from a human, a mouse, a horse, a goat, a sheep, a cow, a primate, or the like.

The term "organoid" as disclosed herein and hereafter may refer to miniaturized structures of cells grown in 3D that, at least partially, resemble microanatomy of an organ in term of structure and function.

The term "spheroid" as disclosed herein and hereafter may refer to cell aggregates that are free-floating, and may be formed by a multicellular mixture of cells within a low-adhesion culture environment.

As disclosed herein and hereafter, a cell culture medium, also referred to as a growth medium, may refer to a liquid, suspension, or gel designed to support cellular growth in an artificial environment, i.e., *in vitro.* There are different types of cell culture media suitable for growing different types of cells. In general, the cell culture media may be broken into two primary categories: natural media and synthetic media. The natural media are those that are derived from tissue extraction or animal body fluids, such as plasma, lymph, and serum. The synthetic media are those created using a variety of organic and inorganic compounds. Moreover, the cell culture medium itself may comprise cells, bodies of cells (e.g., organoids), lipid particles, including natural or engineered (e.g., exosomal microvesicles, vacuoles, micelles or lipid particles of various design, virus particles, nanoparticles, etc.).

As disclosed herein and hereafter, a cell culture apparatus may refer to an apparatus having various wells (e.g., reservoirs, chambers, etc.) connected by flow channels (microchannels) in which fluids (i.e., culture media) will exhibit microfluidic behavior in their flow through the flow channels (microchannels). Such a cell culture apparatus is also referred to as a microfluidic chip in this technical field. The microfluidic cell culture performed by the cell culture apparatus is generally related to cell culture, maintenance, and perturbation in micro-scale fluid volumes. The reasons behind the popularity of the microfluidic cell culture are both economic and scientific. The cell culture apparatuses as disclosed herein may have the advantages of *in vitro* cell culture (high-throughput, parallel experiments, experiments may be done at the discretion of an experimenter, no need for specialized infrastructure and personnel, etc.) with *in vivo* like performance. For example, in mouse models, drugs are really selected for mice, not humans. By using human cells, drugs are screened for humans. Thus, using humanized microfluidic cell and tissue cultures to screen drug candidates reduces pre-clinical trial time and those drugs entering clinical testing are better suited for humans. This may reduce the probability of adverse effects and increase the chance of showing efficacy resulting in less failures in clinical trials.

As disclosed herein and hereafter, each microchannel of the cell culture apparatus is also referred to as a flow channel and may relate to a sub-millimeter-scale channel that has a hydraulic diameter below 1 mm and is used for fluidically connecting the wells of the cell culture apparatus. In other words, the microchannel or flow channel generally denotes a channel configured to pass different fluids, such, for example, as culture media (e.g., cell suspensions), water, reagents, or gels, in micro-scale volumes. The microchannel may be shaped such that it has appropriate flow characteristics (e.g., flow rates) depending on particular applications. For example, the flow channel may have a rectangular, e.g., square, or rounded cross-section, as well as be straight, bended, or tilted towards a required direction.

The apical and basal chambers are separated by a wall comprising through pores formed therein or being free of through pores formed therein.

The wall-based cell culture apparatuses known so far suffer from low throughput and incompatibility with available liquid handling systems (e.g., microtiter plate formats) and imaging systems. Furthermore, the flow control in such apparatuses is often limited to one method and not flexible enough to adapt to different resource settings. On top of that, the cell deposition on the basal (i.e., bottom) surface of the porous wall is possible in the known cell culture apparatuses only with very high cell concentrations, liquid flow control, and by inverting the cell culture apparatus.

The cell culture apparatuses as disclosed in the present disclosure provide a technical solution that allows mitigating or even eliminating the drawbacks of the prior art. In particular, the technical solution disclosed herein provides a cell culture apparatus comprising one or more cell culture modules.

In one aspect is provided a cell culture apparatus comprising:
one or more cell culture modules, each cell culture module of the one or more cell culture modules comprising:
one or more cell culture modules, each cell culture module of the one or more cell culture modules comprising:
   - at least two culture medium reservoirs each having a top part and a bottom part, the top part having an inlet for a culture medium and the bottom part having an outlet for the culture medium;
   - a first chamber arranged between the at least two culture medium reservoirs such that the first chamber and the at least two culture medium reservoirs are aligned with each other in a first direction;
   - a second chamber arranged under the first chamber and aligned with the first chamber in a second direction, the second direction having an angle being more than 0 degrees but 90 degrees or less in respect to the first direction, the second chamber having a bottom part having at least two lateral holes;
   - a wall made of a wall material, the wall being arranged between the first chamber and the second chamber; and
   - at least two flow channels, each of the at least two flow channels being configured to pass a cell culture medium therethrough, and each of the at least two flow channels connecting the outlet of the bottom part of one of the at least two culture medium reservoirs to one of the at least two lateral holes of the bottom part of the second chamber,
wherein the bottom wall of the second chamber is made of an elastic bottom wall material configured to deform in a third direction, the third direction being opposite to the second direction, in response to a force applied on the bottom side of the bottom wall of the second chamber and/or the wall material of the wall is an elastic wall material configured to deform in the second direction in response to a force applied on the first chamber side of the wall.

The one or more cell culture modules of the cell culture apparatus may be arranged adjacent to each other. Each cell culture module comprises two or more culture medium reservoirs connected by two or more flow channels. The flow channels go through a basal chamber (i.e., the second chamber) arranged under an apical chamber (i.e., the first chamber). The apical and basal chambers are separated by a wall comprising through pores formed therein or being free of through pores formed therein. This wall may be a wall that is free of through pores formed therein and, thus, there is no flow communication between the apical side of the wall (i.e., the first chamber side of the wall) and the basal side of the wall, or the wall may comprise one or more through pores formed therein, and thus, the first chamber (i.e., the apical chamber) and the second chamber (i.e., the basal chamber) are in flow communication with each other via the one or more through pores. Therefore, the wall comprising one or more through pores formed therein may be referred in this disclosure as a membrane. In this apparatus configuration, flows of culture media may be perfused and regulated between the culture medium reservoirs in each cell culture module by either placing the apparatus on a rocking platform or connecting the apparatus to a pneumatic pump. Moreover, the apparatus thus configured may cultivate cells of the same or different types on the basal and apical surfaces of the porous walls in the cell culture modules under uniform and controlled *in vitro* conditions. The deposition of the cells on the basal surface of the porous walls may be provided without having to invert the apparatus and to use high concentrations of the cells in the culture medium.

The apparatus as disclosed in the present disclosure may enable one, in response to a force applied on the first chamber side of the wall and/or the bottom side of the bottom wall of the second chamber, to remove efficiently one or more cells, spheroids, and/or organoids present in the second chamber of the apparatus (including the basal side of the wall). The location(s) where one or more cells, spheroids, and/or organoids present in the second chamber (including the basal side of the wall) of the apparatus may be removed from may correspond to the location(s) the force move the wall and/or the bottom wall of the second chamber to in the second and/or third direction in response to the force(s) applied on the bottom side of the bottom wall and/or on the first chamber side of the wall. Thus, the apparatus as disclosed in the present disclosure may be used to investigate, e.g., cell migration and/or wound healing of cell cultures present in the second chamber of the apparatus.

The apparatus comprises one or more cell culture modules. If the apparatus comprises two or more cell culture modules, the two or more cell culture modules may be arranged adjacent to each other.

With this configuration, the cell culture apparatus may cultivate cells of the same or different types on the basal (i.e., bottom) surface of the porous walls in the cell culture modules under uniform and controlled *in vitro* conditions. Moreover, the deposition of the cells on the basal surface of the porous walls may be provided without having to invert the apparatus or to use high concentrations of the cells in the culture medium to cause the cells to bond and cover the wall.

The terms "second direction having an angle being more than 0 degrees but 90 degrees or less in respect to the first direction" as disclosed in the present disclosure refers to a second direction that may be perpendicular to the first direction, or the angle between the second direction and the first direction is more than 0 degrees but less than 90 degrees.

Additionally, or alternatively, the second direction may be approximately perpendicular to the first direction. An angle between the first and the second direction is about 90 degrees, for example.

Additionally, or alternatively, the apparatus further comprises a flow driving unit configured to cause the culture medium to flow, in each cell culture module of the one or more cell culture modules, between the at least two culture medium reservoirs via the at least two flow channels and the second chamber.

Additionally, or alternatively, the elastic bottom wall material of the bottom wall of the second chamber is silicone. Additionally, or alternatively, the elastic bottom wall material of the bottom wall of the second chamber is polydimethylsiloxane, and the thickness of the elastic bottom wall is 8 - 200 µm. Additionally, or alternatively, the bottom wall of the second chamber being configured to deform 12 - 300 µm in the third direction in response to a force applied on the bottom side of the bottom wall of the second chamber.

Additionally, or alternatively, the apparatus comprises a plurality of cell culture modules arranged adjacent to each other.

Additionally, or alternatively, the force is pneumatic pressure, a force applied using a pin, strip, or any protrusion. The deformation of the elastic bottom wall material (and hence, the bottom wall of the second chamber) and/or the elastic wall material (and hence, the wall) may be due to any force applied, as long as the force applied on the bottom side of the bottom wall of the second chamber and/or the first chamber side of the wall deforms the elastic bottom wall material (i.e., the bottom wall) and/or the elastic wall material (i.e., the wall). Preferably the force does not break the bottom wall and the wall.

Additionally, or alternatively, the cell culture module further comprises one or more protrusions attached to and protruding from the wall on the apical side or the basal side of the wall, or attached to and protruding from the upper side of the bottom wall of the second chamber. These embodiments may enable one, in response to a force applied on the first chamber side of the wall and/or the bottom side of the bottom wall of the second chamber, to remove, even more efficiently, one or more cells, spheroids, and/or organoids present in the second chamber of the apparatus. The location(s) where one or more cells, spheroids, and/or organoids present in the second chamber of the apparatus may be removed from may correspond to the location(s) the one or more protrusions move to in the second and/or third direction in response to the force(s) applied on the bottom side of the bottom wall and/or on the first chamber side of the wall. Additionally, or alternatively, if force(s) are applied on the bottom side of the bottom wall of the second chamber or the first chamber side of the wall that does not in combination move the one or more protrusions, one or more cells, spheroids, and/or organoids present in the second chamber of the apparatus may be removed from location(s) corresponding to location(s) the bottom side of the bottom wall of the second chamber or the first chamber side of the wall deforms to. An affected cell culture may be obtained that may be investigated in, e.g., cell migration and/or would healing studies. In addition, depending on the location(s) of the one or more protrusions, the one or more protrusions may remove one or more cells, spheroids, and/or organoids present in the second chamber of the apparatus from specific locations. This opens up new possibilities for, e.g., creating slots (corresponding to the location of the removed one or more cells, spheroids, and/or organoids) for organoid formation on cell culture.

Additionally, or alternatively, the cell culture module further comprises one or more protrusions attached to and protruding from the wall on the apical side of the wall. Having the one or more protrusions attached to and protruding from the wall on the apical side of the wall provides an obstruction-free fluid flow path, keeping a flow laminar, which may be essential for retaining the function of microfluidic cultures.

Additionally, or alternatively, the cell culture module further comprises one or more protrusions attached to and protruding from the wall on the basal side of the wall. These embodiments may enable one, in response to a force applied on the first chamber side of the wall, to remove, even more efficiently, one or more cells, spheroids, and/or organoids present in the second chamber of the apparatus, in particular one or more cells, spheroids, and/or organoids being on the upper side of the bottom wall of the second chamber (i.e., on the upper surface of the bottom wall of the second chamber). The one or more cells, spheroids, and/or organoids may be removed due to the one or more protrusions moving together, due to the deformation of the wall, with the wall. The location(s) where one or more cells, spheroids, and/or organoids present in the second chamber of the apparatus may be removed from may correspond to the location(s) the one or more protrusions move to in the second direction in response to the force(s) applied on the bottom side of the bottom wall. Additionally, or alternatively, if force(s) are applied on the bottom side of the bottom wall of the second chamber (i.e., which may then not move the one or more protrusions), one or more cells, spheroids, and/or organoids present in the second chamber of the apparatus (in particular on the upper side of the bottom wall of the second chamber) may be removed from location(s) corresponding to the location(s) the one or more cells, spheroids, and/or organoids are moved (in the third direction) when the one or more cells, spheroids, and/or organoids are moving with the bottom wall of the second chamber in response to the deformation of the bottom wall. An affected cell culture may be obtained that may be investigated in, e.g., cell migration and/or would healing studies. In addition, depending on the location(s) of the one or more protrusions, the one or more protrusions may remove the one or more cells, spheroids, and/or organoids at specific locations. This opens up new possibilities for, e.g., creating slots (corresponding to the location of the removed one or more cells, spheroids, and/or organoids) for organoid formation on cell culture.

Additionally, or alternatively, the cell culture module further comprises one or more protrusions attached to and protruding from the upper side of the bottom wall of the second chamber. These embodiments may enable one, in response to a force applied on the bottom side of the bottom wall of the second chamber, to remove, even more efficiently, one or more cells, spheroids, and/or organoids present in the second chamber of the apparatus, in particular one or more cells, spheroids, and/or organoids on the basal side of the wall, thereby obtaining an affected cell culture that may be investigated in, e.g., cell migration and/or would healing studies. Again, the location(s) from which one or more cells, spheroids, and/or organoids on the basal side of the wall are removed from corresponds to the location(s) the one or more protrusions move to when moving together with the bottom wall of the second chamber they are attached to. In addition, depending on the location(s) of the one or more protrusions, the one or more protrusions may remove the one or more cells, spheroids, and/or organoids at specific locations. This opens up new possibilities for, e.g., creating slots (corresponding to the location of the removed one or more cells, spheroids, and/or organoids) for organoid formation on cell culture.

Additionally, or alternatively, the one or more protrusions are a plurality of protrusions. In embodiments, the plurality of protrusions are 2-16 protrusions. A plurality of protrusions attached to and protruding from the wall on the apical side or the basal side of the wall, or attached to and protruding from the upper side of the bottom wall of the second chamber may enable one to remove even more cells, spheroids, and/or organoids simultaneously. Additionally, or alternatively, depending on the locations of the plurality of protrusions, one of the plurality of protrusions may first remove, in response to a first force applied on the bottom side of the bottom wall and/or on the first chamber side of the wall, one or more cells, spheroids, and/or organoids from a first location in the second chamber of the apparatus, and after that, in response to a second force applied on the bottom side of the bottom wall and/or on the first chamber side of the wall, one or more cells, spheroids, and/or organoids present in the second chamber of the apparatus may be removed from a second location, and so on. Thus, one or more of the plurality of protrusions may be used individually to remove one or more cells, spheroids, and/or organoids at different locations. This opens up new possibilities for, e.g., creating slots (corresponding to the location of the removed one or more cells, spheroids, and/or organoids) at different time points for formation of different spheroids on cell culture and/or for different sizes of organoids (if the protrusions are of different size) when the created slots are of different shapes and/or sizes.

Additionally, or alternatively, the second chamber further comprises one or more biological material growing means. The one or more biological material growing means may act as means for growing biological material on, such as muscular cells for forming muscular tissue, thus, the one or more biological material growing means may enhance the formation of cells on the one or more biological material growing means.

Additionally, or alternatively, each of the one or more biological material growing means being attached to the upper side of the bottom wall of the second chamber or the basal side of the wall comprising, or being free of, one or more through pores formed therein, provided that if the one or more biological material growing means being attached to the basal side of the wall comprising one or more through pores formed therein, the one or more biological material growing means are attached such that that the first chamber and the second chamber are in flow communication with each other via at least one of the one or more through pores. In embodiments, the bottom wall of the second chamber is made of an elastic bottom wall material configured to deform in a third direction, the third direction being opposite to the second direction, in response to a force applied on the bottom side of the bottom wall of the second chamber.

Additionally, or alternatively, the one or more biological material growing means is two biological material growing means. The two biological material growing means may act as means for growing biological material on, such as muscular cells for forming muscular tissue from one of the two biological material growing means to the other one of the two biological material growing means.

Additionally, or alternatively, each of the one or more biological material growing means are made of a biological material growing means material selected based on at least one type of cells to be grown on the one or more biological material growing means. Apparatuses may be designed for different types of cells to be cultivated on one or more biological material growing means.

Additionally, or alternatively, each of the one or more biological material growing means are made of a biological material growing means material selected from the group consisting of polydimethylsiloxane, polyethylene terephthalate, polystyrene, polycarbonate, a thermal plastic elastomer, polycaprolactone, silicon nitride, copper, hydrogels, and extracellular matrices. Examples of extracellular matrices include, but are not limited to, gelatin, matrigel, collagen, fibronectin, and hyaluronic polymers. Apparatuses may be designed for different types of cells to be cultivated on one or more biological material growing means using these biological material growing means materials.

Additionally, or alternatively, each of the one or more biological material growing means are arranged in the second chamber such that each of the at least two flow channels are in flow communication with each other. By doing so, an efficient flow communication is obtained which may enhance the formation of cells, spheroids, and/or organoids.

Additionally, or alternatively, the wall comprises one or more through pores formed therein and the wall being arranged between the first chamber and the second chamber such that the first chamber and the second chamber are in flow communication with each other via the one or more through pores. These embodiments may enable one to study interactions and wounding mechanisms between cells, spheroids, and/or organoids in the first chamber and cells, spheroids, and/or organoids in the second chamber due to formation of interactions between these cells, spheroids, and/or organoids in the first chamber and in the second chamber.

Additionally, or alternatively, the one or more through pores of the wall has an average pore size falling within a range from 0.2 µm to 200 µm. More specially, the pore sizes may be more or less than sizes of the cells, spheroids, and/or organoids to be deposited on the wall. For example, if the pore size is less than the cell, spheroid, and/or organoid size, then it is possible to prevent the cells, spheroids, and/or organoids from migrating between the basal and apical surfaces of the wall.

Additionally, or alternatively, the wall material is selected based on at least one type of cells, spheroids, and/or organoids to be grown on the wall. By altering the wall material depending on cell, spheroid, and/or organoid types, it is possible to alter different properties of the wall (e.g., chemical and physical properties, such as hydrophilicity/hydrophobicity, stiffness, roughness, cell proliferation, attachment, mobility, survivability, etc.), thereby providing control over how a cell, spheroid, and/or organoid interacts with the wall and other cells, spheroids, and/or organoids. Given this, the wall may be adapted for certain one or more types of cells to be grown thereon.

Additionally, or alternatively, the wall material is selected from the group consisting of polydimethylsiloxane, polyethylene terephthalate, polystyrene, polycarbonate, thermoplastic elastomers, polycaprolactone, silicon nitride, copper, hydrogels, and extracellular matrices. Examples of extracellular matrices include, but are not limited to, gelatin, matrigel, collagen, fibronectin, and hyaluronic polymers.

Additionally, or alternatively, the wall is free of through pores formed therein. These apparatuses may enable one to culture cells in the second chamber and/or in the at least two flow channels. It is to be understood that when the wall is free of through pores formed therein, the wall is arranged between the first chamber and the second chamber such that the first chamber and the second chamber lacks flow communication with each other.

Additionally, or alternatively, the bottom part of the second chamber is made of the elastic bottom part material configured to deform in the third direction, the third direction being opposite to the second direction, in response to a force applied on the bottom side of the bottom wall of the second chamber; the first chamber comprises at least two compartments and at least one compartment wall, wherein the at least two compartments comprise a first compartment and a second compartment, wherein the at least one compartment wall being arranged between the first compartment and the second compartment such that the first compartment and the second compartment are in flow communication with each other; and the wall comprises one or more through pores formed therein and the wall being arranged between the first chamber and the second chamber such that the first chamber and the second chamber are in flow communication with each other via the one or more through pores. At least one of the first compartment and the second compartment may be for receiving one or more cell, spheroid, and/or organoid. The compartments may enable studying interactions between cells, spheroids, and/or organoids compartmentalized in the compartments due to the first compartment and the second compartment being in flow communication with each other. In addition, since the wall comprises one or more through pores formed therein and one or more of the first compartment and second compartment is in flow communication to the second chamber via the through pores, one may study e.g. wounding mechanisms between cells in the second chamber and at least one type of cells, spheroids, and/or organoids in the first and/or second compartment due to formation of interactions between these cells, spheroids, and/or organoids in the second chamber and the at least one type of cells, spheroids, and/or organoids of the first and/or second compartment. For example, the formation of neuromuscular junction (formed between the first and the second compartment) between muscular tissue formed (or provided) in the first compartment and brain organoids formed (or provided) in the second compartment, and vascular cells formed (or provided) in the second chamber may be studied. The vascular cells formed (or provided) in the second chamber may form an endothelial barrier, which may be essential for replicating organ-level function. It is to be understood that in these embodiments the apical side of the wall (i.e., on the first chamber side of the wall) may be free of one or more protrusions attached to and protruding from the wall on the apical side of the wall.

Additionally, or alternatively, the first chamber comprises the at least two compartments comprising the first and the second compartment, and further 1 - 3 compartment(s), and the at least one compartment wall comprises 2-5 compartment walls being arranged between the 3-5 compartments such at least two of the compartments are in flow communication with each other. Three or more types of cells may be provided in the compartments enabling studying interactions between three or more types of cells.

Additionally, or alternatively, the at least one compartment wall being spaced apart from the wall such that the first compartment and the second compartment are in flow communication with each other.

Additionally, or alternatively, the compartment wall being spaced apart from the wall such that the distance between the wall and a bottom part of the compartment wall is between 0 and 20 µm and such that the first compartment and the second compartment are in flow communication with each other. The space between the wall (i.e., on the first chamber side of the wall or the apical side of the wall) and the bottom part of the compartment wall may enable the culturing of one first type of cells, spheroids, and/or organoids in the first compartment of the at least two compartments and one second type of cells, spheroids, and/or organoids in the second compartment of the at least two compartments and the formation of interaction(s) between the first and second type of cells via the space between the wall and the bottom part of the compartment wall. Thus, cell, spheroid, and/or organoid interaction studies may be performed using the apparatuses of these embodiments.

Additionally, or alternatively, the at least one compartment wall comprises one or more compartment wall through pores formed therein and the compartment wall being arranged between the first compartment and the second compartment such that the first compartment and the second compartment are in flow communication with each other via the one or more compartment wall through pores. One or more compartment wall through pores may enable the culturing of one first type of cells, spheroids, and/or organoids in the first compartment of the at least two compartments and one second type of cells, spheroids, and/or organoids in the second compartment of the at least two compartments and the formation of interaction(s) between the first and second type of cells via the one or more compartment wall through pores. Thus, cell, spheroid, and/or organoid interaction studies may be performed using the apparatuses of these embodiments.

Additionally, or alternatively, the at least one compartment wall comprises one or more compartment wall through pores formed therein and the compartment wall being arranged between the first compartment and the second compartment such that the first compartment and the second compartment are in flow communication with each other via the one or more compartment wall through pores, wherein the distance of at least one of the one or more compartment wall through pores from the membrane is less than 20 µm.

Additionally, or alternatively, the one or more compartment wall through pores of the at least one compartment wall has an average pore size falling within a range between 0.2 and 20 µm.

Additionally, or alternatively, the wall material of the wall is an elastic wall material configured to deform in the second direction in response to a force applied on the first chamber side of the wall. These embodiments may enable one to deform the wall in the second direction, i.e., towards the bottom wall of the second chamber.

Additionally, or alternatively, the elastic wall material is selected from the group consisting of polydimethylsiloxane, polyethylene terephthalate, polystyrene, polycarbonate, thermoplastic elastomers, polycaprolactone, silicon nitride, copper, hydrogels, and extracellular matrices. Examples of extracellular matrices include, but are not limited to, gelatin, matrigel, collagen, fibronectin, and hyaluronic polymers.

Additionally, or alternatively, the elastic wall material configured to deform in the second direction in response to a force applied on the first chamber side of the wall is selected from polydimethylsiloxane, and thermoplastic elastomers.

Additionally, or alternatively, the wall material of the wall being configured to deform in the second direction in response to a force applied on the first chamber side of the wall is configured to deform 12 - 300 µm in the second direction in response to the force applied on the first chamber side of the wall.

Additionally, or alternatively, the wall material of the wall being configured to deform in the second direction in response to a force applied on the first chamber side of the wall is configured to deform in the second direction such that it touches the bottom wall of the second chamber in response to a force applied on the first chamber side of the wall.

Additionally, or alternatively, the bottom wall of the second chamber is made of an elastic bottom wall material configured to deform in a third direction, the third direction being opposite to the second direction, in response to a force applied on the bottom side of the bottom wall of the second chamber.

Additionally, or alternatively, the elastic bottom wall material is a transparent elastic bottom wall material.

Additionally, or alternatively, the elastic bottom wall material of the bottom wall of the second chamber is polydimethylsiloxane, and the thickness of the elastic bottom wall is 8 - 200 µm.

Additionally, or alternatively, the bottom wall of the second chamber being configured to deform 12 - 300 µm in the third direction in response to a force applied on the bottom side of the bottom wall of the second chamber.

Additionally, or alternatively, the elastic bottom wall material of the bottom wall of the second chamber configured to deform in the third direction, the third direction being opposite to the second direction, in response to a force applied on the bottom side of the bottom wall of the second chamber is configured to deform in the third direction such that it touches the wall in response to the force applied on the bottom side of the bottom wall of the second chamber.

Additionally, or alternatively, the second chamber of at least one cell culture module of the one or more cell culture modules comprises at least one coating layer being made of a coating material, the at least one coating layer being provided on an inner surface of the second chamber, provided that if the wall comprises one or more through pores formed therein the at least one coating layer being provided on the inner surface of the second chamber such that the one or more through pores of the wall remain open. The at least one coating layer may be a cell adhesion enhancing layer. The cell adhesion enhancing layer may be made of a hydrophilic material such as, but not limited to, type I collagen. With such a coating layer, the cells may attach onto the inner surface of the second chamber more efficiently. Thus, this coating layer allows for less cells to be used in an experiment and more precise deposition of the cells on inner surface of the second chamber. In turn, the smaller number of cells also means less cell death or, in other words, more healthy cells in the cell culture as dead cells tend to release factors promoting apoptosis.

Additionally, or alternatively, the at least one coating layer provided on the inner surface of the second chamber is provided on the upper side of the bottom wall of the second chamber (i.e., on the upper surface of the bottom wall of the second chamber). With such a coating layer, the cells may attach onto the upper side of the bottom wall of the second chamber more efficiently. Thus, this coating layer allows for less cells to be used in an experiment and more precise deposition of the cells on the upper side of the bottom wall of the second chamber.

Additionally, or alternatively, the at least one coating layer provided on the inner surface of the second chamber is provided on the one or more biological material growing means. With such a coating layer, the cells may attach onto the one or more biological material growing means more efficiently. Thus, this coating layer allows for less cells to be used in an experiment and more precise deposition of the cells on the one or more biological material growing means.

Additionally, or alternatively, the coating material of the at least one coating layer is selected based on at least one type of cells to be grown in the second chamber, in particular on the inner surface of the second chamber, on the upper side of the bottom wall of the second chamber, and/or on the one or more biological material growing means.

Additionally, or alternatively, the at least one coating layer is a cell adhesion enhancing layer, the cell adhesion enhancing layer being made of a hydrophilic material, such as, but not limited to, type I collagen.

Additionally, or alternatively, the wall of at least one cell culture module of the one or more cell culture modules comprises at least one coating layer being made of a coating material, the at least one coating layer being provided on the wall, provided that if the wall comprises one or more through pores formed therein the at least one coating layer being provided on the wall such that the one or more through pores of the wall remain open, wherein the coating material of the at least one coating layer is selected based on at least one type of cells to be grown on the wall. With such a coating layer, the cells may attach onto the wall comprising, or being free of, one or more through pores more efficiently. Thus, this coating layer allows for less cells to be used in an experiment and more precise deposition of the cells on the (porous) wall. In turn, the smaller number of cells also means less cell death or, in other words, more healthy cells in the cell culture as dead cells tend to release factors promoting apoptosis.

Additionally, or alternatively, the at least one coating layer provided on the wall is provided on the first chamber side of the wall (i.e., on the apical surface of the wall).

Additionally, or alternatively, the at least one coating layer provided on the wall is provided on the basal side of the wall (i.e., on the basal surface of the wall).

Additionally, or alternatively, the coating material of the at least one coating layer is selected based on at least one type of cells to be grown on the wall.

Additionally, or alternatively, each of the at least two flow channels in at least one cell culture module of the one or more cell culture modules comprises at least one coating layer being made of a coating material, the at least one coating layer being provided on the inner surface of each of the at least two flow channels. The at least one coating layer provided on the inner surface of each of the at least two flow channels may be a cell adhesion enhancing layer and may be made of a hydrophilic material (e.g., type I collagen). By using the adhesion enhancing layer, cell attachment onto the sides of the flow channels (as well as on the sides of the second chamber and the porous wall) may become stronger, cell proliferation may be appropriate to a tissue being modelled, and cell viability may be better. Selecting the correct adhesion enhancing layer also plays a role in the proper communication between cell types. Thus, this coating layer may allow for less cells to be used in an experiment and more precise deposition of the cells on the inner surface of each of the at least two flow channels. In turn, the smaller number of cells also means less cell death or, in other words, more healthy cells in the cell culture as dead cells tend to release factors promoting apoptosis.

Additionally, or alternatively, the first chamber in at least one cell culture module of the one or more cell culture modules is implemented as a bottomless hollow tube. With these first chambers, it is possible to deposit cells, organoids, and/or spheroids on the apical (i.e., top) surface of the wall (membrane). The cells, organoids, and/or spheroids deposited on the apical surface of the wall (membrane) comprising one or more through pores may be of the same or other type compared to the cells deposited on the basal surface of the wall (membrane), in the second chamber, or on the one or more biological material growing means. Thus, the first chamber thus configured may allow one to deposit cells (such as a cell (mono)layer), organoids, and/or spheroids on the basal surface of the wall (membrane), in the second chamber, and/or on the one or more biological material growing means and study the interaction of the cells, organoids, and/or spheroids deposited on the apical surface of the porous wall, alternatively on the apical surface of the porous wall in the one or more compartments, and basal surface of the wall, in the second chamber, and/or on the one or more biological material growing means. Moreover, with such configuration, the first chamber is easier and cheaper to manufacture.

Additionally, or alternatively, the first chamber in at least one cell culture module of the one or more cell culture modules is implemented as a hollow tube having a curved or angled bottom, the curved or angled bottom having at least one first chamber cavity and at least one first chamber hole formed in each of the at least one first chamber cavity, and wherein the bottom wall of the second chamber is made of an elastic bottom wall material configured to deform in a third direction, the third direction being opposite to the second direction, in response to a force applied on the bottom side of the bottom wall of the second chamber. With these first chambers, it is possible to deposit cells on separate portions of the apical surface of the porous wall. This configuration of the first chamber is especially useful for forming similar or different particles under study (e.g., organoids or spheroids) on the apical surface of the porous wall and study their interaction with the cells deposited on the basal surface of the porous wall, in the second chamber, and/or on the one or more biological material growing means in the second chamber, in particular using walls comprising one or more through pores.

Additionally, or alternatively, the curved or angled bottom is coated, from outside, with a cell adhesion enhancing layer such that the at least one first chamber hole of the at least one first chamber cavity remains open, the cell adhesion enhancing layer being made of a hydrophilic material. The hydrophilic material may be e.g., type I collagen. With such a coating layer, the cells may clump outside the bottom of the first chamber and further adhere onto the apical surface of the porous wall. Thus, this coating layer allows for less cells to be used in an experiment and more precise deposition of the cells on the apical surface of the porous wall. In turn, the smaller number of cells also means less cell death or, in other words, more healthy cells in the cell culture as dead cells tend to release factors promoting apoptosis.

Additionally, or alternatively, the second chamber of at least one cell culture module of the one or more cell culture modules has a height falling within a range selected from 50 - 350 µm, 75 - 350 µm, 100 - 350 µm, 125 - 350 µm, 150 - 350, 200 - 350 µm, 250 - 350 µm, 300 - 350 µm, 50 - 300 µm, 50 - 250 µm, 50 - 200 µm, 50 - 150 µm, 50 - 100, and 50 - 75 µm. By using such a second chamber, it is possible to make the cell culture apparatus as disclosed in the present disclosure more compact.

Additionally, or alternatively, the flow driving unit is configured to cause the fluid or culture medium to flow, in each cell culture module of the one or more cell culture modules, between the at least two culture medium reservoirs by:
- supplying a compressed gas or a pressurized air to the inlet of each of the at least two culture medium reservoirs; or
- pipetting the culture medium from one of the at least two culture medium reservoirs to another of the at least two culture medium reservoirs at regular time intervals or based on a level of the culture medium in each of the at least two culture medium reservoirs; or
- periodically rocking the one or more cell culture modules. Thus, unlike current microfluidic cell culture devices, the cell culture apparatus as disclosed in the present disclosure may be compatible with different flow control means, i.e., pneumatic pump-actuated flow control, and pumpless flow control (which is provided by means of gravity as a result of rocking the one or more cell culture modules or by means of culture medium pipetting).

Additionally, or alternatively, each of the at least two culture medium reservoirs in at least one cell culture module of the one or more cell culture modules is implemented as a hollow tube. With such configuration, the culture medium reservoirs are easier and cheaper to manufacture.

Additionally, or alternatively, the bottom part of each of the at least two culture medium reservoirs has a positively tapered profile. By using such a configuration of the culture medium reservoirs, it is possible to cause the whole culture medium contained in the culture medium reservoirs to flow to the bottom towards the second chamber even when the apparatus is tilted, so that no culture medium is left in the culture medium reservoirs. This also helps to reduce the number of cells that is needed to be deposited or grown on the basal surface of the porous wall. Additionally, this configuration of the culture medium reservoirs may allow one to achieve optimal flow rates in each cell culture module.

Additionally, or alternatively, at least one cell culture module of the one or more cell culture modules further comprises at least two electrodes, wherein each of the at least two electrodes is independently arranged in the first chamber, the second chamber, in one or more of the at least two culture medium reservoirs, or the wall, or any combinations thereof. These electrodes may serve different purposes. For example, they may be used to feed electrical pulses to muscle or nerve cells present in the first and/or second chambers or be coupled to other sensors of various types. More specifically, these electrodes may be used to perform real-time Transepithelial/Transendothelial Electrical Resistance (TEER) measurements; in this case, signals may be read out by an external control unit

Additionally, or alternatively, at least one cell culture module of the one or more cell culture modules further comprises a first electrode arranged in the first chamber and a second electrode arranged in the second chamber. These electrodes may serve different purposes. For example, they may be used to feed electrical pulses to muscle or nerve cells present in the first and/or second chambers or be coupled to other sensors of various types. More specifically, these electrodes may be used to perform real-time Transepithelial/Transendothelial Electrical Resistance (TEER) measurements; in this case, signals may be read out by an external control unit.

Additionally, or alternatively, at least one cell culture module of the one or more cell culture modules further comprises a first electrode arranged in one or more of the at least two culture medium reservoirs and a second electrode arranged in the first chamber. This arrangement of the first and second electrodes may be also used to perform the TEER measurements, for example.

Additionally, or alternatively, at least one cell culture module of the one or more cell culture modules further comprises at least two third electrodes arranged in the first chamber. The third electrodes may be arranged on the same wall or on opposite walls inside the first chamber, or one of the at least two third electrodes may be arranged on the wall of the first chamber and another one of the at least two third electrodes may be arranged on the at least one wall arranged between at least two compartments. These electrodes may be used to perform liquid/medium level measurements in the first chamber. These arrangements of the first and second electrodes may be also used to perform the TEER measurements, for example.

Additionally, or alternatively, at least one cell culture module of the one or more cell culture modules further comprises at least two fourth electrodes imbedded into the wall. These electrodes may be used, for example, to provide different stimulus signals to the cells deposited on the apical and/or basal surface of the wall and/or the surface of the upper side of the bottom wall of the second chamber and/or on the one or more biological material growing means of the second chamber.

Additionally, or alternatively, at least one cell culture module of the one or more cell culture modules further comprises at least one of an oxygen sensor, a pH sensor and a CO₂ sensor in the first chamber or the second chamber. These sensors enable real-time measurements of parameters crucial for cell behavior.

Additionally, or alternatively, the bottom part of the second chamber being arranged higher than the bottom part of each of the at least two culture medium reservoirs.

Additionally, or alternatively, each of the at least two flow channels extends at least partly at a tilting angle to the first direction, the tilting angle falling within a range of 5 degrees to 85 degrees. With such titled flow channels, it is possible to achieve appropriate flow characteristics and, therefore, improve cell delivery to the second chamber.

Additionally, or alternatively, the one or more through pores of the wall has an average pore size falling within a range from 0.2 µm to 200 µm. By varying the average pore size within this range, it is possible to study the behavior of cells of different sizes and types, e.g. neuronal cells and muscular cells, that are deposited on one or each of the apical and basal surfaces of the wall, the surface of the upper side of the bottom wall of the second chamber and/or on the one or more biological material growing means of the second chamber.

Additionally, or alternatively, each of the at least two flow channels in at least one cell culture module of the one or more cell culture modules has a variable channel height and/or a variable channel width. This may allow achieving better retainment of the laminar flow of the culture medium in the flow channels.

Additionally, or alternatively, the variable channel width and/or the variable channel height gradually increases towards the second chamber. This may allow achieving better retainment of the laminar flow of the culture medium in the flow channels. With this configuration of the flow channels, it may also be possible to improve flow characteristics in each cell culture module, thereby improving the cell and/or cell culture medium delivery to the second chamber and, consequently, the cell formation at, and/or cell attachment to, the basal surface of wall, the surface of the upper side of the bottom wall of the second chamber, and/or the one or more biological material growing means of the second chamber may be improved. In addition, cell and/or cell culture medium delivery to the first chamber via the one or more through pores, if present, may be improved, thus cell spheroid, and/or organoid formation in the first chamber may be improved.

Additionally, or alternatively, each of the at least two flow channels in at least one cell culture module of the one or more cell culture modules comprises a first channel portion and a second channel portion, the first channel portion extending from the outlet of the bottom part of one of the at least two culture medium reservoirs and being parallel to the first direction, the second channel portion connecting the first channel portion to one of the at least two lateral holes of the second chamber, the second channel portion being tilted relative to the first direction. With this configuration of the flow channels, it is possible to improve flow characteristics in each cell culture module, thereby improving the cell delivery to the second chamber.

In a second aspect is provided a cell culture incubator comprising:
a cell culture apparatus as disclosed in the present disclosure; and
a pipetting station configured to feed the fluid or culture medium to each of the at least two culture medium reservoirs in each cell culture module of the one or more cell culture modules. It is to be understood that the "cell culture apparatus as disclosed in the present disclosure" as used herein and hereafter may refer to a cell culture apparatus according to the first aspect. With this configuration, the cell culture incubator may cultivate cells, spheroids, and/ or organoids of the same or different types in the cell culture apparatus of the one or more cell culture modules under unform and controlled *in vitro* conditions. Cell culture apparatuses comprising a wall coated with at least one coating layer on the basal surface of the wall may provide deposition of cells on the basal surface of the walls, comprising, or being free of, through pores, without having to invert the cell culture apparatus or to use high concentrations of the cells in the culture medium to cause the cells to bond and cover the basal surface of wall.

In a third aspect is provided a method for affecting a cell culture, the method comprising:
(a) providing a cell culture apparatus as disclosed in the present disclosure, wherein each second chamber of each cell culture module of the one or more cell culture modules comprises a cell culture comprising a plurality of cells of a first type of cells;
(b) applying one or more first force
   i) on one or more first locations on the bottom side of the bottom wall of the second chamber such that the bottom wall of the second chamber deforms in the third direction; and/or
   ii) on one or more second locations on the first chamber side of the wall such that the wall deforms in the second direction,
thereby removing at least one cell from the plurality of cells of the first type of cells of the cell culture, and forming a first affected cell culture. It is to be understood that the "cell culture apparatus as disclosed in the present disclosure" as used herein and hereafter may refer to a cell culture apparatus according to the first aspect. By this method, it is possible to creating slots (corresponding to the location of the removed at least one cell of the plurality of cells of the first type of cells of the cell culture) corresponding to a wound on the cell culture, and to analyze, e.g., cell migration after the wound has been created (if the thus affected cell culture is cultivated). It is to be understood that applying one or more (first) force may additionally, or alternatively, to removing at least one cell, damage at least one cell. The cells of the first type of cells may be one type of cells or different types of cells. It is to be understood that "plurality of cells" may be two or more cells, three or more cells, typically more than 100 cells, more than 1000 cells, 100 - 10⁶ cells, or even more cells.

Additionally, or alternatively, (a) providing a cell culture apparatus comprises:
- providing a cell culture apparatus as disclosed in the present disclosure;
- providing a first cell culture medium to one of the at least two medium reservoirs of each cell culture module of the one or more cell culture modules, wherein the first cell culture medium comprises a first type of cells;
- causing the first cell culture medium to flow from said one of the at least two culture medium reservoirs via the second chamber to the rest of the at least two culture medium reservoirs in each cell culture module of the one or more cell culture modules for a first predefined flow time period, the first predefined flow time period being selected based on the first cell culture medium; and
- incubating the cell culture apparatus for a first predefined incubating time period, thereby forming a cell culture comprising a plurality of cells of a first type of cells in each second chamber of each cell culture module of the one or more cell culture modules of the cell culture apparatus, the first predefined incubating time period being selected based on the first type of cells.

Alternatively, a method for affecting a cell culture, the method comprising:
(a) providing a cell culture apparatus as disclosed in the present disclosure;
   - providing a fifth cell culture medium via the first chamber, or via the first compartment or the second compartment of the first chamber, of each cell culture module of the one or more cell culture modules towards the first chamber side of the wall, wherein the fifth cell culture medium comprises a fifth type of cells;
   - incubating the cell culture apparatus for a fifth predefined incubating time period, thereby forming a cell culture comprising a plurality of cells of a fifth type of cells in each first chamber or in each first or second compartment of each cell culture module of the one or more cell culture modules of the cell culture apparatus, the fifth predefined incubating time period being selected based on the fifth type of cells;
   - providing a first cell culture medium to one of the at least two medium reservoirs of each cell culture module of the one or more cell culture modules, wherein the first cell culture medium comprises a first type of cells;
   - causing the first cell culture medium to flow from said one of the at least two culture medium reservoirs via the second chamber to the rest of the at least two culture medium reservoirs in each cell culture module of the one or more cell culture modules for a first predefined flow time period, the first predefined flow time period being selected based on the first cell culture medium; and
   - incubating the cell culture apparatus for a first predefined incubating time period, thereby forming a cell culture comprising a plurality of cells of a first type of cells in each second chamber of each cell culture module of the one or more cell culture modules of the cell culture apparatus, the first predefined incubating time period being selected based on the first type of cells;
(b) applying one or more first force
   i) on one or more first locations on the bottom side of the bottom wall of the second chamber such that the bottom wall of the second chamber deforms in the third direction,
thereby removing at least one cell from the plurality of cells of the first type of cells of the cell culture and forming a first affected cell culture. It is to be understood that applying one or more (first) force may additionally, or alternatively, to removing at least one cell, damage at least one cell. The causing the first cell culture medium to flow may be caused by a flow driving unit.

Additionally, or alternatively, the method further comprises:
(c) providing a second cell culture medium comprising a second type of cells to one of the at least two medium reservoirs of each cell culture module of the one or more cell culture modules;
(d) causing the second culture medium to flow from said one of the at least two culture medium reservoirs via the second chamber to the rest of the at least two culture medium reservoirs in each cell culture module of the one or more cell culture modules for a second predefined flow time period, the second predefined flow time period being selected based on the second culture medium; and
(e) incubating the cell culture apparatus for a second predefined incubating time period thereby forming a first cultured affected cell culture, the second predefined incubating time period being selected based on the first and second type of cells. By so doing, it is possible to cultivate cells, spheroids, and/or organoids of the same or different types (i.e., the second type of cells being the same as, or different to, the first type of cells) in the slots (corresponding to the location of the removed at least one of the plurality of cells of the one or more type of cells of the cell culture) created. Cells, spheroids, and/or organoids may be formed under unform and controlled *in vitro* conditions in the slots. This opens up new possibilities for, e.g., cells, spheroids, and/or organoids formation and/or cell migration studies.

Additionally, or alternatively, wherein, in (d), the causing the first cell culture medium to flow is caused by a flow driving unit.

Additionally, or alternatively, the second type of cells being same as, or different to, the first type of cells, and, in (e), the second predefined incubating time period being selected based on the first and second type of cells.

Additionally, or alternatively, the second type of cells being different to the first type of cells, and, in (e), the second predefined incubating time period being selected based on the first and second type of cells. E.g., organoids may be formed from the second cell culture medium of (c) comprising the second type of cells in the created slots of a thick cell culture of the first type of cells.

Additionally, or alternatively, the second type of cells being same as the first type of cells, and, in (e), the second predefined incubating time period being selected based on the first and second type of cells. E.g., wound healing and/or cell migration may be studied using these embodiments.

Additionally, or alternatively, the method further comprises:
- applying one or more second force
   i) on one or more third locations on the bottom side of the bottom wall of the second chamber, the one or more third locations being different from the one or more first locations, such that the bottom wall of the second chamber deforms in the third direction; and/or
   ii) on one or more fourth locations on the first chamber side of the wall, the one or more fourth locations being different from the one or more second locations, such that the wall deforms in the second direction,
thereby removing at least one cell from the first affected cell culture, or from the first cultured affected cell culture, and forming a second affected cell culture or a second affected cultured cell culture.

Slots may be formed at location(s) where one or more cells, spheroids, and/or organoids present in the second chamber of the apparatus may have been removed from. Said location(s) may correspond to the location(s) the bottom wall of the second chamber deforms to in the third direction and/or the wall deforms to in the second direction (both or either of the bottom wall and the wall may have one or more protrusions, thus where the one or more protrusions move to) in response to the one or more second force applied on the bottom side of the bottom wall and/or on the first chamber side of the wall. Additionally, or alternatively, if force(s) are applied on the bottom side of the bottom wall of the second chamber or the first chamber side of the wall that does not in combination move the one or more protrusions, one or more cells, spheroids, and/or organoids present in the second chamber of the apparatus may be removed from location(s) corresponding to location(s) the bottom side of the bottom wall of the second chamber or the first chamber side of the wall deforms to. A second affected cell culture or a second affected cultured cell culture may be obtained that may be investigated in, e.g., cell migration and/or would healing studies. In addition, depending on the location(s) of the optional one or more protrusions, the one or more protrusions may remove one or more cells, spheroids, and/or organoids present in the second chamber of the apparatus from specific locations. This opens up new possibilities for, e.g., creating slots (corresponding to the location of the removed one or more cells, spheroids, and/or organoids) for organoid formation on cell culture.

Additionally, or alternatively, the method further comprises:
- applying one or more second force
   i) on one or more third locations on the bottom side of the bottom wall of the second chamber, the one or more third locations being different from the one or more first locations, such that the bottom wall of the second chamber deforms in the third direction; and/or
   ii) on one or more fourth locations on the first chamber side of the wall, the one or more fourth locations being different from the one or more second locations, such that the wall deforms in the second direction,
   thereby removing at least one cell from the first affected cell culture, or from the first cultured affected cell culture, and forming a second affected cell culture or a second affected cultured cell culture;
- providing a third cell culture medium comprising a third type of cells to one of the at least two medium reservoirs of each cell culture module of the one or more cell culture modules;
- causing the third culture medium to flow from said one of the at least two culture medium reservoirs via the second chamber to the rest of the at least two culture medium reservoirs in each cell culture module of the one or more cell culture modules for a third predefined flow time period, the third predefined flow time period being selected based on the third culture medium; and
- incubating the cell culture apparatus for a third predefined incubating time period, the third predefined incubating time period being selected based on the first type of cells and/or the third type of cells.

By doing so, one or more cells are removed from the first affected cell culture, or from the first cultured affected cell culture, and the slots(s) thus formed (corresponding to the location(s) from where cells are removed) may be occupied with cells of the second and/or third cell culture medium (i.e., of a second and/or third type of cells). Studying and/or analyzing cell migration and/or wound healing may be performed. In addition, the slot(s) may be for cell, spheroid, and/or organoid formation from the second and/or third type of cells. The causing the third culture medium to flow may be caused by a flow driving unit.

Additionally, or alternatively, the third type of cells are the same as the first and second type of cells. Studying of wound healing may be performed using these methods.

Additionally, or alternatively, the first, second, and third type of cells are different. Cell, spheroid, and/or organoid formation of two different types from the cells of the second and third type of cells may be obtained in the slot(s) created in the first affected cell culture and/or the second affected cell culture and/or the second affected cultured cell culture. E.g., cells, spheroids, and/or organoids of a first type may be formed from the second type of cells in the slot(s) created in the first affected cell culture, and cells, spheroids, and/or organoids of a second type may be formed from the third type of cells in the slot(s) created in the second affected cultured cell culture. It is to be understood that different combinations of cell, spheroid, and/or organoid formation in slot(s) created may be obtained.

Additionally, or alternatively, the third type of cells is different to the first and second type of cells, the second type of cells is the same as the first type of cells. Wound healing and/or cell migration studies of the first and second type of cells may be studied and cell, spheroid, and/or organoid formation of the third type of cells may be obtained in the slots created in the second affected cultured cell culture.

Additionally, or alternatively, the causing the first and/or second and/or third culture medium to flow from the one of the at least two culture medium reservoirs via the second chamber to the rest of the at least two culture medium reservoirs in each cell culture module of the one or more cell culture modules comprises applying a positive pressure to the at least two culture medium reservoirs in each cell culture module of the one or more cell culture modules for the first and/or second and/or third predefined flow time period. By so doing, it is possible to provide a proper culture medium flow in each cell culture module, thereby improving cell cultivation on the porous walls in each cell culture module.

Additionally, or alternatively, the causing the first and/or second and/or third culture medium to flow from the one of the at least two culture medium reservoirs to the rest of the at least two culture medium reservoirs in each cell culture module of the one or more cell culture modules comprises applying a positive pressure to the one of the at least two culture medium reservoirs in each cell culture module of the one or more cell culture modules for the first and/or second and/or third predefined flow time period, while capping the rest of the at least two culture medium reservoirs. By so doing, it is possible to provide a proper culture medium flow in each cell culture module, thereby improving cell cultivation on the porous walls in each cell culture module.

Additionally, or alternatively, the at least two culture medium reservoirs in each cell culture module of the one or more cell culture modules comprises a first culture medium reservoir and a second culture medium reservoir, and wherein causing the first and/or second and/ or third second culture medium to flow from the one of the at least two culture medium reservoirs via the second chamber to the rest of the at least two culture medium reservoirs in each cell culture module of the one or more cell culture modules comprises:
- applying a first positive pressure to the first culture medium reservoir in each cell culture module of the one or more cell culture modules for the first and/or second and/or third predefined time interval, while capping the second culture medium reservoir; and
- applying a second positive pressure to the second culture medium reservoir in each cell culture module of the one or more cell culture modules for the first and/or second and/or third predefined time interval, while capping the first culture medium reservoir. By so doing, it is possible to provide a proper culture medium flow in each cell culture module, thereby improving cell cultivation on the porous walls in each cell culture module.

Additionally, or alternatively, the method further comprises:
- providing a fourth cell culture medium comprising a fourth type of cells via the first chamber of each cell culture module of the one or more cell culture modules towards the first chamber side of the wall; and
- incubating the cell culture apparatus for a fourth predefined incubating time period, the fourth predefined incubating time period being selected based on the fourth type of cells,
wherein the wall of each cell culture module of the one or more cell culture modules comprises one or more through pores formed therein and the wall being arranged between the first chamber and the second chamber such that the first chamber and the second chamber are in flow communication with each other via the one or more through pores, and wherein any of the applying one or more force(s) is on the one or more first and/or third locations on the bottom side of the bottom wall of the second chamber. These embodiments may enable one to cultivate a fourth type of cells (e.g. neuronal cells) on the first chamber side of the wall (i.e., the apical side of the wall) in the first chamber, and to cultivate a first type of cells (e.g. muscular cells for forming muscular tissue), and optionally a second and/or third type of cells (e.g., as second type of cells vascular cells), in the second chamber, in particular on the upper side of the bottom wall of the second chamber and/or on the one or more biological material growing means and/or on the basal (i.e., bottom) side of the wall. In addition, e.g., wounding healing mechanisms and/or neuromuscular junction formation may be studied using the cells formed in these methods. "Any of the applying one or more force(s)" may refer to applying one or more first and/or second force(s). The providing a fourth cell culture medium and the incubating the cell culture apparatus (i.e., culturing at least the fourth type of cells) may be performed before applying one or more first force on the one or more first locations on the bottom side of the bottom wall of the second chamber. By so doing, it is possible to cultivate the fourth type of cells in the first chamber before slot(s) are being created in the second chamber (due to any force(s) applied), thus the fourth type of cells may form spheroids and/or organoids on the first chamber side of the wall before the slots have been made. Different cell co-cultures may be generated and studying and/or analyzing cell migration and/or wound healing may be performed.

Additionally, or alternatively, the fourth type of cells are different to the first type of cells, and if second and/or third type of cells are present, the fourth type of cells are different to the second and/or third type of cells.

Additionally, or alternatively, the cell culture comprising a plurality of cells of a first type of cells is, at least partially, on the one or more biological material growing means, preferably the one or more biological material growing means is two biological material growing means and the first type of cells is, at least partially, on and between the two biological material growing means, the two biological material growing means being spaced apart, and the fourth type of cells is different from the first type of cells. E.g., the first type of cells is muscular cells, and the fourth type of cells is neuronal cells.

Additionally, or alternatively, the method further comprises:
- providing a fourth cell culture medium comprising a fourth type of cells via a first compartment of the first chamber of each cell culture module of the one or more cell culture modules towards the first chamber side of the wall;
- providing a fifth cell culture medium comprising a fifth type of cells via a second compartment of the first chamber of each cell culture module of the one or more cell culture modules towards the first chamber side of the wall; and
- incubating the cell culture apparatus for a fifth predefined incubating time period, the fifth predefined incubating time period being selected based on the fourth and fifth type of cells,

wherein the wall of each cell culture module of the one or more cell culture modules comprises one or more through pores formed therein and the wall being arranged between the first chamber and the second chamber such that the first chamber and the second chamber are in flow communication with each other via the one or more through pores,
wherein the first chamber comprises the at least one wall, and wherein the at least one wall being arranged between the first compartment and the second compartment such that the first compartment and the second compartment are in flow communication with each other, and wherein any of the applying one or more force(s) is on the one or more first and/or third locations on the bottom side of the bottom wall of the second chamber.

These embodiments may enable one to cultivate a fourth type of cells (e.g., neuronal cells) on the first chamber side of the wall (i.e., the apical side of the wall) in the first compartment of the first chamber, a fifth type of cells (e.g., muscular cells) on the first chamber side of the wall in the second compartment of the first chamber (i.e., the apical side of the wall), and to cultivate a first and/or second and/or third type of cells (e.g., a first type of cells being vascular cells) in the second chamber, in particular on the upper side of the bottom wall of the second chamber and/or on the one or more biological material growing means and/or on the basal (i.e., bottom) side of the wall. E.g., a neuromuscular junction between neuronal cells in the first compartment and muscular cells in the second compartment may be formed after the incubation. E.g. vascular cells being formed (or provided) in the second chamber may form an endothelial barrier, which may be essential for replicating organ-level function. In addition, e.g., wounding healing mechanisms and/or neuromuscular junction formation may be studied using the cells formed in these methods. The providing a fourth cell culture medium, fifth cell culture medium, and the incubating the cell culture apparatus (i.e., culturing at least the fourth and fifth type of cells) may be performed before applying one or more first force and/or second force (on the one or more first and/or third locations on the bottom side of the bottom wall of the second chamber). By so doing, it is possible to cultivate the fourth and fifth type of cells in the first and second compartments before one or more slots are being created in the second chamber (due to the one or more force(s) applied), thus the fourth and fifth type of cells may form spheroids and/or organoids on the first chamber side of the wall (in the first and second compartments) before the slots have been made. Different cell co-cultures may be generated and studying and/or analyzing cell migration and/or wound healing may be performed. Additionally, or alternatively, one of the fourth and fifth cell culture media may be excluded and, thus, only the fourth cell culture medium or the fifth cell culture media may be provided in the first or second compartment, respectively.

Additionally, or alternatively, the fourth and/or fifth type of cells are different to the first type of cells, and if second and/or third type of cells are present, the fourth and/or fifth type of cells are different to the second and/or third type of cells. In embodiments, the fourth and fifth type of cells are different to the first type of cells, and if second and/or third type of cells are present, the fourth and fifth type of cells are different to the second and third type of cells. By doing so, one may obtain cells, organoids, and/or spheres of different types in each of the first compartment, the second compartment, and second chamber.

Additionally, or alternatively, any of the force(s) is pneumatic pressure, a force applied using a pin, strip, or any protrusion. "Any of the forces" as used herein and hereafter may refer to any of the one or more first and second forces. The deformation of the elastic bottom wall material (and hence, the bottom wall of the second chamber) and/or the elastic wall material (and hence, the wall) may be due to any force applied, as long as the force applied on the bottom side of the bottom wall of the second chamber and/or the first chamber side of the wall deforms the elastic bottom wall material (i.e., the bottom wall) and/or the elastic wall material (i.e., the wall). Preferably the force does not break the bottom wall and the wall.

Additionally, or alternatively, the method further comprises, after the causing the first culture medium to flow and/or the causing the second culture medium to flow and/or causing the third culture medium to flow,
- placing the cell culture apparatus in an inverted position; and
- incubating the cell culture apparatus in the inverted position for a sixth predefined incubating time period, the sixth predefined incubating time period being selected based on the culture medium present in the second chamber.

By so doing, it is possible to cultivate cells of the same or different types on the porous walls of the cell culture modules under unform and controlled *in vitro* conditions. Although the deposition of the cells on the basal surface of the porous walls is provided by inverting the cell culture apparatus, these methods do not require high concentrations of the cells to be used in the culture medium to cause the cells to bond and cover the wall, in particular when the basal surface of the wall is coated with a coating layer selected based on at least one type of cells to be grown on the wall. It is to be understood that in these methods, if there are cells, spheroids, and/or organoids present in any of the first chamber and the at least two compartments (first and second compartments), the placing the cell culture apparatus in an inverted position and incubating the cell culture apparatus in the inverted position may not be performed.

Additionally, or alternatively, the cell culture, the first, the second, the third, the fourth, and the fifth cell culture medium each independently comprises a cell type selected from the group consisting of astrocytes, brain organoids, brain spheroids, neuronal cells, vascular endothelial cells, and muscular cells. Examples of muscular cells include, but are not limited to, smooth muscle cells, cardiac muscle cells, and skeletal muscle cells. Examples of brain organoids include, but are not limited to, human brain, mid-brain, and cortical organoids. Examples of vascular endothelial cells include, but are not limited to, brain vascular endothelial cells, in particular human brain vascular endothelial cells or human primary brain microvascular endothelial cells (HPBMEC).

Additionally, or alternatively, the first, second, third, fourth, and/or fifth type of cells is each independently selected from the group consisting of astrocytes, such as human or mouse astrocytes; brain organoids, such as human brain, mid-brain, and cortical organoids; brain spheroids, such as human brain, mid-brain, and cortical spheroids, neuronal cells, endothelial cells, such as vascular endothelial cells, in particular human vascular endothelial cells or human primary brain microvascular endothelial cells (HPBMEC); epithelial cells, musculoskeletal cells, induced pluripotent stem cells (IPSC) derived or differentiated cells, and muscular cells.

Additionally, or alternatively, in (a), plurality of cells of the first type of cells is endothelial cells, epithelial cells, musculoskeletal cells or induced pluripotent stem cells (IPSC) derived or differentiated cells. These first type of cells may be in a form of a monolayer, hydrogel embedded cell suspension, or thick layered cell layer. Examples of first type of cells include, but are not limited to, vascular endothelial cells, such as human vascular endothelial cells or human primary brain microvascular endothelial cells (HPBMEC); or muscular cells.

Additionally, or alternatively, the second type of cells is endothelial cells, epithelial cells, musculoskeletal cells or induced pluripotent stem cells (IPSC) derived cells or -differentiated cells. These second type of cells may be in a form of a monolayer, hydrogel embedded cell suspension, or thick layered cell layer. Examples of second type of cells include, but are not limited to, vascular endothelial cells, such as human vascular endothelial cells or human primary brain microvascular endothelial cells (HPBMEC); or muscular cells.

Additionally, or alternatively, the third type of cells is endothelial cells, epithelial cells, musculoskeletal cells or induced pluripotent stem cells (IPSC) derived cells or -differentiated cells. These third type of cells may be in a form of a monolayer, hydrogel embedded cell suspension, or thick layered cell layer. Examples of third type of cells include, but are not limited to, vascular endothelial cells, such as human vascular endothelial cells or human primary brain microvascular endothelial cells (HPBMEC); or muscular cells.

Additionally, or alternatively, the fourth type of cells is endothelial cells, epithelial cells, musculoskeletal cells, induced pluripotent stem cells (IPSC) derived cells or -differentiated cells, or astrocytes, such as human or mouse astrocytes. These fourth type of cells may be in a form of a monolayer, hydrogel embedded cell suspension, or thick layered cell layer. Examples of fourth type of cells include, but are not limited to, vascular endothelial cells, such as human vascular endothelial cells or human primary brain microvascular endothelial cells (HPBMEC); or muscular cells.

Additionally, or alternatively, the fifth type of cells is endothelial cells, epithelial cells, musculoskeletal cells, induced pluripotent stem cells (IPSC) derived cells or -differentiated cells, or astrocytes, such as human or mouse astrocytes. These fifth type of cells may be in a form of a monolayer, hydrogel embedded cell suspension, or thick layered cell layer. Examples of fifth type of cells include, but are not limited to, vascular endothelial cells, such as human vascular endothelial cells or human primary brain microvascular endothelial cells (HPBMEC); or muscular cells.

Additionally, or alternatively, the first type of cells is selected from neuronal cells, muscular cells, and vascular endothelial cells; the second type of cells is selected from neuronal cells, muscular cells, and vascular endothelial cells; the third type of cells is selected from neuronal cells, muscular cells, and vascular endothelial cells; the fourth type of cells is selected from neuronal cells, and muscular cells; and the fifth type of cells is selected from neuronal cells, and muscular cells.

Additionally, or alternatively, the plurality of cells of the first type of cells comprised in the cell culture is endothelial cells and the endothelial cells being in the form of a (mono)layer in each second chamber of each cell culture module of the one or more cell culture modules; the second type of cells is pericytes and the second cell culture medium further comprises matrigel, wherein the second cell culture medium comprising pericytes and matrigel forms a (thin) layer of pericytes and matrigel on the (mono)layer of endothelial cells;
the third type of cells is osteoblasts, wherein the osteoblasts form a bone base on the (thin) layer of pericytes and matrigel;
the fourth type of cells are muscular cells, wherein the fourth cell culture medium comprises a (high) concentration of the muscular cells and matrigel or collagen; and
the fifth type of cells is neurons.

In a fourth aspect is provided a use of a cell culture apparatus as disclosed in the present disclosure for analyzing cell cultures, in methods for cell cultivation, and for affecting cell cultures.

The analyzing cell cultures includes, but is not limited to, for analysis in protein assays, cell viability assays, genomic and proteomic analysis, metabolic assays, and with imaging systems.

Additionally, or alternatively, the analyzing cell cultures is selected from for analysis in protein assays, cell viability assays, genomic and proteomic analysis, metabolic assays, and with imaging systems.

FIG. 1 shows a block diagram of a cell culture apparatus 100 in accordance with exemplary embodiments. The apparatus 100 is intended for the above-mentioned microfluidic cell culture. As shown in FIG. 1, the apparatus 100 may comprise a cell culture plate 102 and a flow driving unit 104 coupled to the cell culture plate 102. The apparatus 100 comprises one or more cell culture modules 106. The cell culture plate 102 may comprise the one or more cell culture modules 106 and may be configured to fit the standard 96, 384, or 1536 microtiter plates (American National Standards Institute (ANSI), having standardized footprint dimensions, plate height, flange dimensions, chamfers, side wall rigidity, and well positions). The apparatus 100 may further comprise a flow driving unit 104 configured to cause a culture medium to flow in each cell culture module 106, as will be described below in more detail. It should be noted that the number, arrangement, and interconnection of the constructive elements constituting the apparatus 100, which are shown in FIG. 1, are not intended to be any limitation of the present invention, but merely used to provide a general idea of how the constructive elements may be implemented within the apparatus 100. For example, the cell culture plate 102 may be replaced with two or more cell culture plates, and the flow driving unit 104 may be replaced with two or more flow driving units each configured to control a culture medium flow in each cell culture module 106.

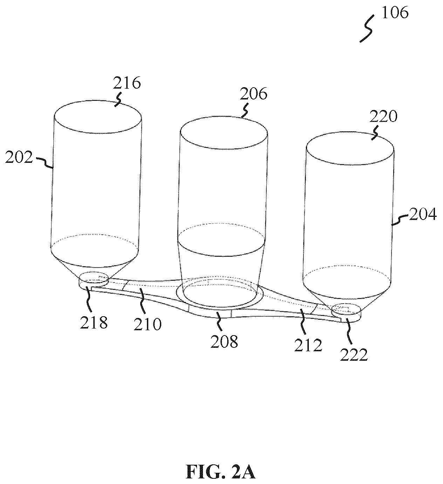

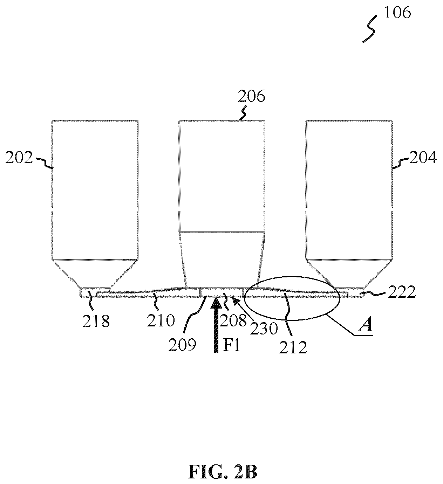

FIGs. 2A-2C show different schematic views of the cell culture module 106 included in the apparatus 100 in accordance with exemplary embodiments. More specifically, FIG. 2A shows a schematic perspective view of the cell culture module 106, FIG. 2B shows a schematic side view of the cell culture module 106, and FIG. 2C shows a schematic top view of the cell culture module 106. As shown in FIGs. 2A-2C, the cell culture module 106 comprises a first culture medium reservoir 202, a second culture medium reservoir 204, a first (apical or top) chamber 206, a second (basal or bottom) chamber 208, a first flow channel 210, a second flow channel 212, and a wall 214. It should be again noted that the present disclosure is not limited to the shown number, arrangement, and interconnection of the constructive elements of the cell culture module 106. In embodiments, there may be more than two medium culture reservoirs, thereby leading to more than two flow channels. In other embodiments, there may be more than two apical chambers and more than two basal chambers between the two culture reservoirs, thereby also increasing the number of the flow channels connecting the two culture reservoirs through the apical chambers and the basal chambers.

The first culture medium reservoir 202 has a top part with an inlet 216 for the culture medium and a bottom part with an outlet 218 for the culture medium. Similarly, the second culture medium reservoir 204 has a top part with an inlet 220 for the culture medium and a bottom part with an outlet 222 for the culture medium. The first and second culture medium reservoirs 202 and 204 may be implemented as identical hollow tubes. Although FIGs. 2A and 2C show that each of the first and second culture medium reservoirs 202 and 204 has a circular cross-section, this should not be construed as any limitation of the present disclosure; in some embodiments, any other cross-sectional shapes, such as polygonal, oval, etc., are possible, if required and depending on particular applications. Moreover, the bottom part of each of the first and second culture medium reservoirs 202 and 204 may have a different profile, for example, a positively tapered profile as shown in FIGs. 2A and 2B.

The first chamber 206 is arranged between the first and second medium culture reservoirs 202 and 204 such that the first chamber 206 and the first and second culture medium reservoirs 202 and 204 are aligned with each other in a first (horizontal) direction. The first chamber 206 may be implemented as a bottomless hollow tube, for example, with a positively tapered profile (as shown in FIGs. 2A and 2B) or with a uniform cross-section.

Again, the shown circular cross-section of the first chamber 206 is for illustrative purposes only and should not be considered as any limitation of the present disclosure.

The second chamber 208 is arranged under the first chamber 206 and aligned with the first chamber 206 in a second (vertical) direction. The second chamber 208 may have a cross-section corresponding to the cross-section of the first chamber 206. The second chamber 208 has a bottom part having two or more lateral holes (not shown in FIGs. 2A-2C). As shown in FIGs. 2A-2C, the second chamber 208 has dimensions significantly smaller than the first chamber 206. For example, if the first chamber has a height of about 7 mm, then the height of the second chamber 208 may fall within a range from 50 µm to 150 µm (at height values higher than 150 µm, the second chamber 208 may lose microfluidic properties). Such height values of the first and second chambers 206 and 208 (in addition to the heights of the reservoirs 202 and 204) make the cell culture module 106 more compact, thereby reducing the total dimensions of the apparatus 100. The second chamber 208 has a bottom wall 209 (shown in FIG. 2B) that may be made of an elastic bottom wall material configured to deform in a third direction, the third direction being opposite to the second direction, in response to a force applied on the bottom side 230 of the bottom wall 209 of the second chamber 208 according to the arrow F1 (see FIG. 2B). If the bottom wall 209 is not made of an elastic bottom wall material configured to deform in the third direction in response to a force applied on the bottom side 230, the wall material of the wall 214 (see FIG. 2C) is an elastic wall material configured to deform in the second direction in response to a force applied on the first chamber side 231 (not shown) of the wall 214 according to the arrow F2 (not shown). Both the bottom wall 209 may be made of an elastic bottom wall material configured to deform in a third direction, the third direction being opposite to the second direction, in response to a force applied on the bottom side 230 of the bottom wall 209 of the second chamber 208 according to the arrow F1, and the bottom wall 209 may be made of an elastic bottom wall material configured to deform in the third direction, the wall material of the wall 214 is an elastic wall material configured to deform in the second direction in response to a force applied on the first chamber side 231 of the wall 214 according to the arrow F2.

The wall 214 (see FIG. 2C) is arranged between the first chamber 206 and the second chamber 208 (the second chamber 208 not shown in FIG. 2C). In embodiments, the wall 214 is arranged between the first chamber 206 and the second chamber 208 (not shown), the wall 214 may have one or more through pores (see FIG. 2C) and the wall 214 is arranged between the first chamber 206 and the second chamber 208 such that the first chamber 206 and the second chamber 208 are in flow communication with each other via the through pores of the wall 214. A wall 214 having one or more through pores formed therein may be referred herein and hereafter as a membrane (having one or more through pores formed therein). The wall (or membrane) 214 may be made of a wall material of silicone, plastic, protein, natural polymers, artificial polymers (e.g., polyester (PET)), metallic polymers or carbohydrates (e.g., cellulose), and may be formed by using one of the following methods: lithography, stamping, casting, electrospinning or *in situ* polymerization. The through pores may have different cross-sectional shapes, such, for example, as triangular, rectangular, square, oval, or polygonal (e.g., hexagonal). The polygonal cross-section shape of the through pores may refer to a convex or concave polygon. In embodiments, the through pores may have different cross-sectional shapes, such as any combination of two or more of the above-mentioned cross-sectional shapes (e.g., the combination of circular and square cross-section shapes). Furthermore, each of the through pores may have a pore size varying within a predefined range of values. For example, the pore size may vary from 0.2 µm to 200 µm, or from 0.2 µm to 10 µm. In general, the predefined range of values for the pore size (as well as the spacing between the through pores) is selected based on certain cells to be deposited on the wall 214 (being a membrane since it has one or more through pores). More specially, the pore sizes may be more or less than sizes of the cells to be deposited on the wall and the one or more through pores of the wall has an average pore size falling within a range from 0.2 µm to 200 µm. For example, if the pore size is less than the cell size, then it is possible to prevent the cells from migrating between the basal and apical surfaces of the wall (membrane) 214. As explained above, the wall material of the wall 214 may be an elastic wall material configured to deform in the second direction in response to a force applied on the first chamber side 231 (not shown) of the wall 214 according to the arrow F2 (not shown). If the wall material of the wall 214 is not made of an elastic wall material configured to deform in the second direction in response to a force applied on the bottom side 230, the second chamber has a bottom wall 209 (shown in FIG. 2B) made of an elastic bottom wall material configured to deform in the third direction, the third direction being opposite to the second direction, in response to a force applied on the bottom side 230 of the bottom wall 209 of the second chamber 208 according to the arrow F1 (see FIG. 2B).

The first and second flow channels 210 and 212 are microchannels providing the passage of the culture medium or fluids between the first and second culture medium reservoirs 202 and 204 through the second chamber 208. In particular, the first flow channel 210 connects the outlet 218 of the bottom part of the first culture medium reservoir 202 to one or more of the lateral holes arranged on the left side of the bottom part of the second chamber 208. The second flow channel 212 connects the outlet 222 of the bottom part of the second culture medium reservoir 204 to one or more of the lateral holes arranged on the right side of the bottom part of the second chamber 208. Each of the first and second flow channels 210 and 212 may have a longitudinal section and a cross-section which allow one to achieve required flow characteristics (e.g., an appropriate flow rate). For example, each of the first and second flow channels 210 and 212 may a variable channel height and a variable channel width which increase (e.g., gradually) towards the second chamber 208.

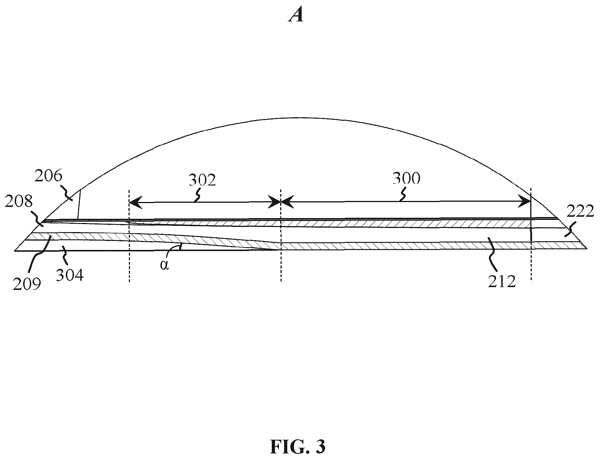

FIG. 3 shows a sectional view of the second flow channel 212 of the cell culture module 106, as taken within the area delimited by oval A in FIG. 2B, in accordance with exemplary embodiments. It should be noted that the first flow channel 210 may have a similar first channel portion 300 and a second channel portion 302 as the second flow channel, only presented as a mirror image. As shown in FIG. 3, the second flow channel 212 comprises a first channel portion 300 and a second channel portion 302. The first channel portion 300 extends from the outlet 222 of the bottom part of the second culture medium reservoir 204 and is parallel to the first (horizontal) direction. The second channel portion 302 connects the first channel portion 300 to the lateral hole(s) (not shown in FIG. 3) arranged on the right side of the second chamber 208. The second channel portion is tilted up (i.e., at a tilting angle α to the first direction) such that the bottom part of the second chamber 208 is arranged higher than the bottom part of the second culture medium reservoir 204 (i.e., higher than the end of the outlet 222). This tilt of the second channel portion 302 is provided by a small "hill" 304 which supports the second chamber 208. The tilting angle α of the second channel portion 302 relative to the first (horizontal) direction preferably falls within a range of 5 degrees to 85 degrees to provide appropriate flow characteristics. In embodiments, each of the first and second flow channels 210 and 212 may be fully tilted up at the tilting angle α, starting from the outlet of the corresponding culture medium reservoir (i.e., there may be no first channel portion parallel to the first (horizontal) direction). The wall 214 is not shown in FIG. 3.

In embodiments, each of the second chamber 208, the first flow channel 210, and the second flow channel 212 may be coated, from inside, with a cell adhesion enhancing layer. The cell adhesion enhancing layer may be made of a hydrophilic material. Some non-restrictive examples of the hydrophilic material may include Matrigel, fibronectin, hyaluronic acid, different types of collagen (e.g., a type I collagen which may be suitable for creating a blood-brain barrier cell culture model based on the apparatus 100), laminins, tenascin, elastane, nanocellulose with a host of different molecules. Such cell adhesion enhancing layers allow for less cells to be used in an experiment and more precise deposition of the cells on the basal surface of the wall 214.

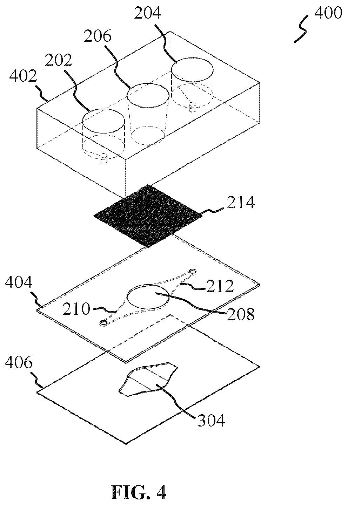

FIG. 4 shows a schematic exploded view of a layered structure 400 that represents one possible implementation example of the cell culture module 106. As shown in FIG. 4, the layered structure 400 comprises a first layer 402, a second layer 404, and a third layer 406. The first layer 402 comprises the first and second culture medium reservoirs 202 and 204, as well as the first chamber 206. The second layer 404 comprises the second chamber 208 arranged exactly under the wall 214, as well as the flow channels 210 and 212. The wall 214 is arranged between the first chamber 206 and the second chamber 208. In embodiments, the wall 214 comprises one or more through pores formed therein to provide the fluid communication between the first chamber 206 and the second chamber 208 via the through pores. Moreover, the inlets of the flow channels 210 and 212 are arranged exactly under the outlets 218 and 222 (not shown in FIG. 4) of the culture medium reservoirs 202 and 204, respectively, to provide the fluid communication between the reservoirs 202 and 204 and the flow channels 210 and 212, respectively. The third layer 406 comprises the small "hill" 304 which is formed such that the appropriate tilting angle α of each of the flow channels 210 and 212 is provided. It should be noted that each of the first layer 402, the second layer 404, and the third layer 406 may be made of room-temperature-vulcanizing (RTV) silicone (e.g., RTV615), plastic (PET, PS, PP, PC, PMMA and similar), glass or polydimethylsiloxane (PDMS). Moreover, the layers 402-406 may be attached to each other in the order shown in FIG. 4 by using adhesive or plasma bonding. The cell culture modules 106 thus configured may be attached to each other by the using the same adhesive or plasma bonding to implement the cell culture plate 102 of the apparatus 100. The first chamber side 231 and the bottom side 230 of the bottom wall 209 are not shown in FIG. 4.

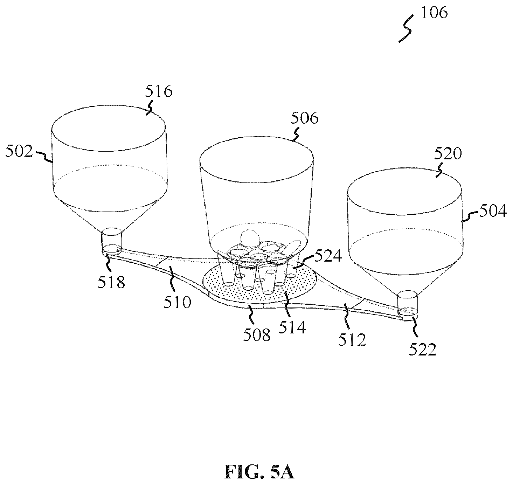

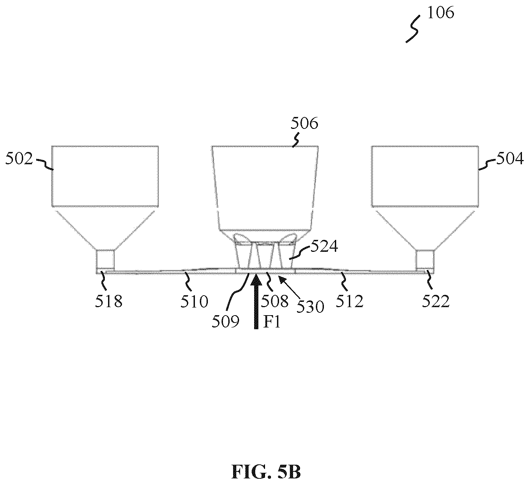

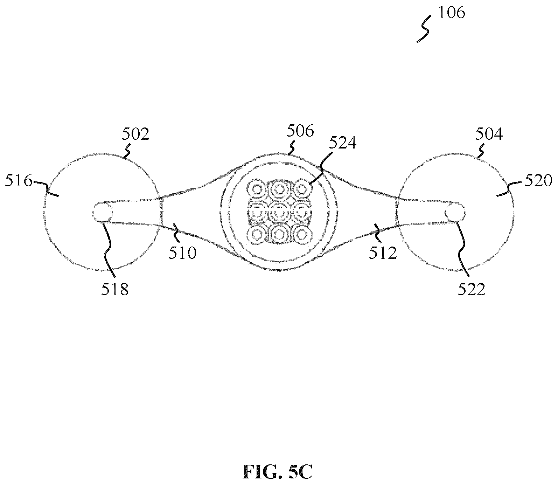

FIGs. 5A-5C show different schematic views of a cell culture module 106 included in the apparatus 100 in accordance with exemplary embodiments. More specifically, FIG. 5A shows a schematic perspective view of the cell culture module 106, FIG. 5B shows a schematic side view of the cell culture module 106, and FIG. 5C shows a schematic top view of the cell culture module 106. As shown in FIGs. 5A-5C, the cell culture module 106 comprises a first culture medium reservoir 502, a second culture medium reservoir 504, a first (apical or top) chamber 506, a second (basal or bottom) chamber 508, a first flow channel 510, a second flow channel 512, and a wall 514. The first culture medium reservoir 502 has a top part with an inlet 516 for the culture medium and a bottom part with an outlet 518 for the culture medium. Similarly, the second culture medium reservoir 504 has a top part with an inlet 520 for the culture medium and a bottom part with an outlet 522 for the culture medium. It should be again noted that the present disclosure is not limited to the shown number, arrangement, and interconnection of the constructive elements of the cell culture module 106. In general, the first culture medium reservoir 502, the second culture medium reservoir 504, the second chamber 508, the first flow channel 510, the second flow channel 512, and the wall 514 may be implemented in the same or similar manner as the first culture medium reservoir 202, the second culture medium reservoir 204, the second chamber 208, the first flow channel 210, the second flow channel 212, and the wall 214, respectively.

At the same time, unlike the first chamber 206, the first chamber 506 may be implemented as a hollow tube having a curved or angled bottom, the wall 514 has one or more through pores formed therein and the wall 514 being arranged between the first chamber 506 and the second chamber 508 such that the first chamber 506 and the second chamber 508 are in flow communication with each other via the one or more through pores, and the second chamber has a bottom wall 509 (see FIG. 5B) made of an elastic bottom wall material configured to deform in the third direction, the third direction being opposite to the second direction, in response to a force applied on the bottom side 530 of the bottom wall 509 of the second chamber 508 according to the arrow F1 (see FIG. 5B). As shown in FIGs 5A-5C, the curved or angled bottom of the first chamber 506 comprises nine chamber cavities 524 each provided with one or more chamber holes, so that the first chamber 506 and the second chamber 508 are in fluid communication via the through pores of the wall 514. It should be obvious to those skilled in the art that the shown number, shape, and arrangement of the chamber cavities 524 are for illustrative purposes only and should not be construed as any limitation of the present disclosure. For example, there may be a single chamber cavity 524 with a single chamber hole. In general, the configuration of the chamber cavity or cavities 524 depends on particular applications (e.g., a number of organoids and/spheroids to be formed on the apical surface of the wall 514. In embodiments, the curved or angled bottom of the first chamber 506 may be coated, from outside, with a cell adhesion enhancing layer such that the holes of the chamber cavities 524 remains open. The cell adhesion enhancing layer may be the same as the one mentioned earlier in accordance with the embodiments of the cell culture module 106.

Referring back to FIG. 1, the optional flow driving unit 104 is configured to cause the culture medium to flow, in each cell culture module 106 of the cell culture plate 102, between the first and second culture medium reservoirs 202 (502) and 204 (504). In embodiments, the flow driving unit 104 may be a rocking platform that is configured to periodically rock the cell culture plate 102, thereby providing the culture medium flow. In another embodiments, the flow driving unit 104 may be a pneumatic pump that is configured to supply a compressed gas or a pressurized air to the inlet of each of the first and second culture medium reservoirs 202 (502) and 204 (504) in each cell culture module 106 (of the cell culture plate 102), thereby causing the culture medium flow therein. In another embodiments, the flow driving unit 104 may be configured to cause the culture medium flow in each cell culture module 106 (of the cell culture plate 102) by pipetting the culture medium from the first culture medium reservoir 202 (502) to the second culture medium reservoir 204 (504), or vice versa, at regular time intervals or based on a level of the culture medium in each of the reservoirs. Thus, unlike the current microfluidic cell culture devices, the apparatus 100 may be compatible with at least three different flow control methods which are indicated above.

In embodiments, the apparatus 100 may further comprise a variety of electrodes. These electrodes may serve different purposes, and their arrangement inside the apparatus 100 depends on which purpose they are used for. In embodiments, one or more cell culture modules (of the cell culture plate 102) may comprise a first electrode arranged in the first chamber 206 (506) and a second electrode arranged in the second chamber 208 (508). In another embodiment, the first electrode may be arranged in the first culture medium reservoir 202 (502) and/or the second culture medium reservoir 204 (504) in one or more culture modules 106, while the second electrode may be arranged in the first chamber 206 (506) of the cell culture module(s) 106. These embodiments with the first and second electrodes may be, for example, used to perform real-time Transepithelial/Transendothelial Electrical Resistance (TEER) measurements. In this case, the first and second electrodes may be connected to an external control unit.

Additionally, or alternatively, the apparatus 100 may further comprise two or more third electrodes arranged in the first chamber 206 (506) of one or more cell culture modules 106. These third electrodes may be arranged on the chamber walls for measuring a fluid level inside the first chamber 206 (506). In this case, the third electrodes may either be in direct contact with the fluid/culture medium to determine the fluid level by performing conductivity measurements, or be embedded into the chamber walls (e.g., at a distance of 0.5 mm - 2 mm from each other) to determine the fluid level by measuring inductive or capacitive changes in the third electrodes. At the same time, the two third electrodes may be arranged opposite to each other such that one of the two third electrodes is used to issue an ultrasound wave and another of the two third electrodes is used to receive the ultrasound wave passed through the interior of the first chamber 206 (506) (these ultrasound measurements may be performed just like radar measurements).

Additionally, or alternatively, the apparatus 100 may further comprise two or more fourth electrodes embedded into the wall 214 (514) of one or more cell culture modules 106. These fourth electrodes may be used to provide different stimulus signals to cells deposited on the apical and/or basal surface of the porous wall.

In embodiments, the first chamber 206 (506) or the second chamber 208 (508) of one or more cell culture modules 106 (of the cell culture plate 102) may further comprise an oxygen sensor, a pH sensor, a CO₂ sensor, or any combination thereof. These sensors may be used to enable real-time measurements of different parameters crucial for cell behavior.

FIGs. 6A-6G show schematic sectional side views of a cell culture module 106 (as taken along line A-A in FIG. 2C) included in the apparatus 100 in accordance with exemplary embodiments. As shown in FIGs. 6A-6G, the cell culture module 106 of the exemplary embodiments of FIGs. 6A-6G comprises a first culture medium reservoir 202, a second culture medium reservoir 204, a first (apical or top) chamber 206, a second (basal or bottom) chamber 208, a first flow channel 210, a second flow channel 212, and a wall 214 having one or more through pores 240 formed therein (FIGs. 6A-6C) or being free of through pores formed therein (FIGs. 6D-6G) (FIG. 2C shows through pores of the wall 214). The first culture medium reservoir 202 has a top part with an inlet 216 for the culture medium and a bottom part with an outlet 218 for the culture medium. Similarly, the second culture medium reservoir 204 has a top part with an inlet 220 for the culture medium and a bottom part with an outlet 222 for the culture medium. The second chamber 208 has a bottom wall 209 (509) made of an elastic bottom wall material configured to deform in a third direction, the third direction being opposite to the second direction, in response to a force according to the arrow F1 applied on the bottom side 230 (530) of the bottom wall 209 of the second chamber 208 and/or the wall material of the wall 214 is an elastic wall material configured to deform in the second direction in response to a force according to the arrow F2 applied on the first chamber side 231 (531) of the wall 214. It should be again noted that the present disclosure is not limited to the shown number, arrangement, and interconnection of the constructive elements of the cell culture module 106. In general, the first culture medium reservoir 202, the second culture medium reservoir 204, the second chamber 208, the first flow channel 210, the second flow channel 212, and the wall 214 of the cell culture module 106 may be implemented in the same or similar manner as explained for the cell culture module 106 of FIGs. 2A-2C (the first culture medium reservoir 202, the second culture medium reservoir 204, the second chamber 208, the first flow channel 210, the second flow channel 212, and the wall 214, respectively).

More specifically, FIG. 6A shows a schematic sectional side view of the cell culture module 106 (as taken along line A-A in FIG. 2C) in accordance with exemplary embodiments, wherein the wall 214 comprises one or more through pores 240 formed therein and the wall being arranged between the first chamber 206 and the second chamber 208 such that the first chamber 206 and the second chamber 208 are in flow communication with each other via the one or more through pores 240. The second chamber 208 has a bottom wall 209 (509) made of an elastic bottom wall material configured to deform in a third direction, the third direction being opposite to the second direction, in response to a force according to the arrow F1 applied on the bottom side 230 (530) of the bottom wall 209 of the second chamber 208. The upper side 232 of the bottom wall 209 of the second chamber 208, the basal side 233 of the wall 214 (514), and the first chamber side 231 (531) (i.e., the apical side) of the wall 214 (514) is shown. The cell culture module 106 may further comprise one or more protrusions 280 (not shown) attached to and protruding from the wall 209 on the apical side (i.e., the first chamber side) 231 or the basal side 233 of the wall 214, or attached to and protruding from the upper side 232 of the bottom wall 209 of the second chamber 208. Additionally, or alternatively, the second chamber 208 may further comprises one or more biological material growing means 270 (not shown but see, e.g., FIG. 6B) arranged such that a force applied on the bottom side 230 of the bottom wall 209 of the second chamber 208 causes the bottom wall 209 of the second chamber 208 to deform.

More specifically, FIG. 6B shows a schematic sectional side view of the cell culture module 106 (as taken along line A-A in FIG. 2C) in accordance with exemplary embodiments, wherein the wall 214 comprises one or more through pores 240 formed therein and the wall 214 being arranged between the first chamber 206 and the second chamber 208 such that the first chamber 206 and the second chamber 208 are in flow communication with each other via the one or more through pores 240. The second chamber 208 has a bottom wall 209 (509) made of an elastic bottom wall material configured to deform in a third direction, the third direction being opposite to the second direction, in response to a force according to the arrow F1 applied on the bottom side 230 (530) of the bottom wall of the second chamber 208. Furthermore, the second chamber 208 has one or more biological material growing means 270 arranged such that a force according to the arrow F1 applied on the bottom side 230 of the bottom wall 209 of the second chamber 208 causes the bottom wall 209 of the second chamber 208 to deform. In embodiments, the one or more biological material growing means 270 are two biological material growing means 270 arranged such that a force according to the arrow F1 applied on the bottom side 230 of the bottom wall 209 of the second chamber 208 causes the bottom wall 209 of the second chamber 208 to deform between the two biological material growing means 270. As shown in the exemplary embodiment of FIG. 6B, two biological material growing means 270 are attached to the upper side 232 of the bottom wall 209 of the second chamber 298 such that each of the at least two flow channels 210, 212 are in flow communication with each other. Each of the one or more biological material growing means 270 may be attached to the basal side 233 of the wall 214 such that that the first chamber 206 and the second chamber 208 are in flow communication with each other via at least one of the one or more through pores 240 and such that each of the at least two flow channels 210, 212 are in flow communication with each other.

More specifically, FIG. 6C shows a schematic sectional side view of the cell culture module 106 (as taken along line A-A in FIG. 2C) in accordance with exemplary embodiments, wherein the wall 214 comprises one or more through pores 240 formed therein and the wall 214 being arranged between the first chamber 206 and the second chamber 208 such that the first chamber 206 and the second chamber 208 are in flow communication with each other via the one or more through pores 240. The second chamber 208 has a bottom wall 209 (509) made of an elastic bottom wall material configured to deform in a third direction, the third direction being opposite to the second direction, in response to a force according to the arrow F1 applied on the bottom side 230 (530) of the bottom wall of the second chamber 208. The first chamber 206 has at least two compartments and at least one compartment wall 260 (one is shown in FIG. 6C), wherein the at least two compartments comprise a first compartment 251 and a second compartment 252, wherein the at least one compartment wall 260 is arranged between the first compartment and the second compartment such that the first compartment 251 and the second compartment 252 are in flow communication with each other. It should be noted that FIG. 2C does not show at least one compartment wall 260 in the first chamber 206. The at least one compartment wall 260 is arranged between the first compartment and the second compartment such that the first compartment 251 and the second compartment 252 are in flow communication with each other. FIG. 6C shows embodiments, wherein the at least one compartment wall 260 being spaced apart from the wall 214 such that the first compartment 251 and the second compartment 252 are in flow communication with each other via the space 262. Additionally, or alternatively, the at least one compartment wall 260 comprises one or more wall through pores formed therein (not shown) and the compartment wall 260 being arranged between the first compartment 251 and the second compartment 252 such that the first compartment 251 and the second compartment 252 are in flow communication with each other via the one or more wall through pores. As disclosed for the exemplary embodiments of the cell culture modules 106 of FIGs. 6A-6B, the cell culture module 106 shown in FIG. 6C may also comprise one or more biological material growing means 270 in the second chamber 208 (not shown) and/or one or more protrusions 280 (not shown) attached to and protruding from basal side 233 of the wall 214, or attached to and protruding from the upper side 232 of the bottom wall 209 of the second chamber 208. If the one or more biological material growing means 270 are attached to the basal side 233 of the wall 214 comprising one or more through pores 240 formed therein, the one or more biological material growing means 270 are attached such that that the first chamber 206 and the second chamber 208 are in flow communication with each other via at least one of the one or more through pores 240.

More specifically, FIG. 6D shows a schematic sectional side view of the cell culture module 106 (as taken along line A-A in FIG. 2C) in accordance with exemplary embodiments. In contrast to the cell culture modules 106 shown in FIGs. 6A-6C, the wall 214 of the cell culture module 106 shown in FIG. 6D is free of one or more through pores 240 (it should be noted that FIG. 2C shows one or more through pores). I.e., the first chamber 206 and the second chamber 208 are free of a flow communication with each other. The second chamber 208 has a bottom wall 209 (509) made of an elastic bottom wall material configured to deform in a third direction, the third direction being opposite to the second direction, in response to a force according to the arrow F1 applied on the bottom side 230 (530) of the bottom wall 209 of the second chamber 208 and/or the wall material of the wall 214 is an elastic wall material configured to deform in the second direction in response to a force according to the arrow F2 applied on the first chamber side 231 (531) of the wall 214. As disclosed for the exemplary embodiments of the cell culture modules 106 of FIGs. 6A-6C, the cell culture module 106 shown in FIG. 6D may also comprise one or more protrusions 280 (not shown) attached to and protruding from the wall 209 on the apical side (i.e., the first chamber side) 231 or the basal side 233 of the wall 214, or attached to and protruding from the upper side 232 of the bottom wall 209 of the second chamber 208. The wall 214 may comprise one or more through pores 240 formed therein and the wall 214 being arranged between the first chamber 206 and the second chamber 208 such that the first chamber 206 and the second chamber 208 are in flow communication with each other via the one or more through pores 240.

More specifically, FIG. 6E shows a schematic sectional side view of the cell culture module 106 (as taken along line A-A in FIG. 2C) in accordance with exemplary embodiments, wherein second chamber 208 of comprises one or more biological material growing means 270. The second chamber 208 has a bottom wall 209 (509) made of an elastic bottom wall material configured to deform in a third direction, the third direction being opposite to the second direction, in response to a force according to the arrow F1 applied on the bottom side 230 (530) of the bottom wall 209 of the second chamber 208 and/or the wall material of the wall 214 is an elastic wall material configured to deform in the second direction in response to a force according to the arrow F2 applied on the first chamber side 231 of the wall 214. As shown in FIG. 6E, two biological material growing means 270 are attached to the upper side 232 of the bottom wall 209 of the second chamber 208 such that each of the at least two flow channels 210, 212 are in flow communication with each other. Furthermore, the two biological material growing means 270 are attached to the upper side 232 of the bottom wall 209 such that bottom wall 209 (509) deforms in a third direction, the third direction being opposite to the second direction, in response to a force according to the arrow F1 applied on the bottom side 230 (530) of the bottom wall 209 of the second chamber 208 and/or the wall 214 deforms in the second direction in response to a force according to the arrow F2 applied on the first chamber side 231 of the wall 214, preferably the bottom wall 209 (509) and/or the wall 214 deforms between the two biological material growing means 270 (in response to the force according to the arrow F1 and/or the force according to the arrow F2). The one or more biological material growing means 270 may be attached to the basal side 233 of the wall 209 such that each of the at least two flow channels 210, 212 are in flow communication with each other (not shown). The wall 214 is free of one or more through pores 240 (it should be noted that FIG. 2C shows one or more through pores). Additionally, or alternatively, as disclosed for the exemplary embodiments of the cell culture modules 106 of FIGs. 6A-6D, the cell culture module 106 shown in FIG. 6E may also comprise one or more protrusions 280 (not shown) attached to and protruding from the wall 209 on the apical side (i.e., the first chamber side) 231 or the basal side 233 of the wall 214, or attached to and protruding from the upper side 232 of the bottom wall 209 of the second chamber 208. The wall 214 may comprise one or more through pores 240 formed therein and the wall 214 being arranged between the first chamber 206 and the second chamber 208 such that the first chamber 206 and the second chamber 208 are in flow communication with each other via the one or more through pores 240.

More specifically, FIG. 6F shows a schematic sectional side view of the cell culture module 106 (as taken along line A-A in FIG. 2C) in accordance with exemplary embodiments, wherein the cell culture module 106 has one or more protrusions 280 attached to and protruding from the basal side 233 of the wall 214. Additionally, or alternatively, the cell culture module 106 may comprise one or more protrusions 280 attached to and protruding from the wall 209 on the apical side (i.e., the first chamber side) 231 and/or on or more protrusions 280 attached to and protruding from the upper side 232 of the bottom wall 209 of the second chamber 208 (protrusion(s) not shown). The wall 214 is free of one or more through pores 240 (it should be noted that FIG. 2C shows one or more through pores). The second chamber 208 has a bottom wall 209 (509) made of an elastic bottom wall material configured to deform in a third direction, the third direction being opposite to the second direction, in response to a force according to the arrow F1 applied on the bottom side 230 (530) of the bottom wall 209 of the second chamber 208 and/or the wall material of the wall 214 is an elastic wall material configured to deform in the second direction in response to a force according to the arrow F2 applied on the first chamber side 231 of the wall 214. The wall 214 may comprise one or more through pores 240 formed therein and the wall 214 being arranged between the first chamber 206 and the second chamber 208 such that the first chamber 206 and the second chamber 208 are in flow communication with each other via the one or more through pores 240.

More specifically, FIG. 6G shows a schematic sectional side view of the cell culture module 106 (as taken along line A-A in FIG. 2C) in accordance with exemplary embodiments, wherein the cell culture module 106 has one or more protrusions 280 (only one is shown) attached to and protruding from the basal side 233 of the wall 214. Additionally, or alternatively, the cell culture module 106 may comprise one or more protrusions 280 attached to and protruding from the wall 209 on the apical side (i.e., the first chamber side) 231 and/or on or more protrusions 280 attached to and protruding from the upper side 232 of the bottom wall 209 of the second chamber 208 (not shown). The wall 214 is free of one or more through pores 240 (it should be noted that FIG. 2C shows one or more through pores). The second chamber 208 has a bottom wall 209 (509) made of an elastic bottom wall material configured to deform in a third direction, the third direction being opposite to the second direction, in response to a force according to the arrow F1 applied on the bottom side 230 (530) of the bottom wall 209 of the second chamber 208 and/or the wall material of the wall 214 is an elastic wall material configured to deform in the second direction in response to a force according to the arrow F2 applied on the first chamber side 231 of the wall 214. As shown in FIG. 6G, two biological material growing means 270 are attached to the upper side 232 of the bottom wall 209 of the second chamber 208 such that each of the at least two flow channels 210, 212 are in flow communication with each other. Furthermore, the two biological material growing means 270 are attached to the upper side 232 of the bottom wall 209 such that bottom wall 209 (509) deforms in a third direction, the third direction being opposite to the second direction, in response to a force according to the arrow F1 applied on the bottom side 230 (530) of the bottom wall 209 of the second chamber 208 and/or the wall 214 deforms in the second direction in response to a force according to the arrow F2 applied on the first chamber side 231 of the wall 214, preferably the bottom wall 209 (509) and/or the wall 214 deforms between the two biological material growing means 270 (in response to the force according to the arrow F1 and/or the force according to the arrow F2). The one or more biological material growing means 270 may be attached to the basal side 233 of the wall 209 such that each of the at least two flow channels 210, 212 are in flow communication with each other (not shown). The wall 214 may comprise one or more through pores 240 formed therein and the wall 214 being arranged between the first chamber 206 and the second chamber 208 such that the first chamber 206 and the second chamber 208 are in flow communication with each other via the one or more through pores 240.

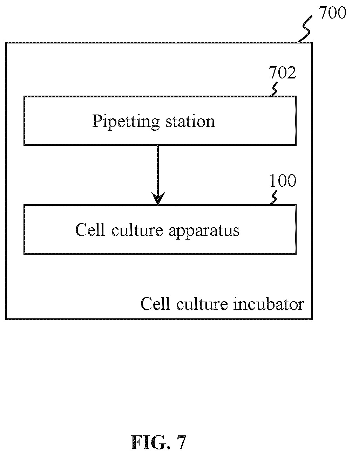

FIG. 7 shows a block diagram of a cell culture incubator 700 in accordance with one exemplary embodiment. The cell culture incubator 700 comprises the cell culture apparatus 100, and a pipetting station 702 coupled to the apparatus 100. The apparatus 100 may comprise a cell culture plate 102 and a flow driving unit 104 coupled to the cell culture plate 102. More specifically, the pipetting station 702 is configured to feed the culture medium to each of the first and second culture medium reservoirs 202 (502) and 204 (504) in each cell culture module 106 (of the cell culture plate 102). The pipetting station 702 is well-known in this technical field, for which reason its description is omitted herein.

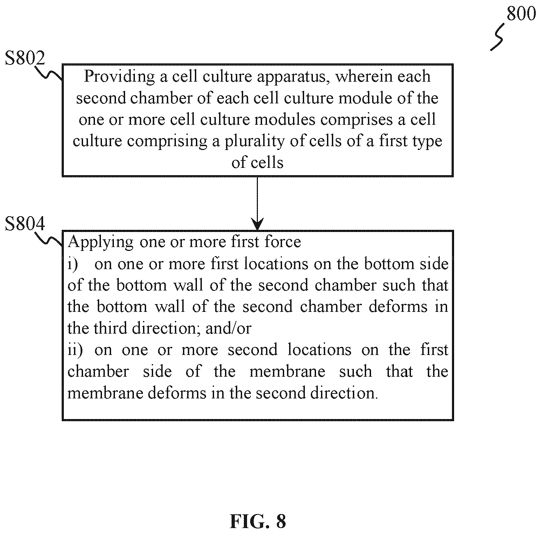

FIG. 8 shows a flowchart of a method 800 for affecting a cell culture in accordance with exemplary embodiments. The method 800 starts with a step S802, in which a cell culture apparatus 100 is provided, wherein each second chamber 208, 508 of each cell culture module of the one or more cell culture modules 106 comprises a cell culture comprising a plurality of cells of a first type of cells. In step S804 is applied one or more first force
i) on one or more first locations on the bottom side 230, 530 of the bottom wall 209, 509 of the second chamber 208, 508 such that the bottom wall 209, 509 of the second chamber 208, 508 deforms in the third direction; and/or
ii) on one or more second locations on the first chamber side 231 of the wall 214 such that the wall 214 deforms in the second direction,
thereby removing at least one cell from the plurality of cells of the first type of cells of the cell culture and forming a first affected cell culture. It is to be understood that applying one or more (first) force may additionally, or alternatively, to removing at least one cell, damage at least one cell.

Alternatively, step S802 comprises substeps S802(a)-S802(d):
substep S802(a): providing a cell culture apparatus 100;
substep S802(b): providing a first cell culture medium to one of the at least two medium reservoirs 202, 204; 502, 504 of each cell culture module of the one or more cell culture modules 106, wherein the first cell culture medium comprises a first type of cells;
substep S802(c): causing the first cell culture medium to flow from said one of the at least two culture medium reservoirs 202, 204; 502, 504 via the second chamber 208, 508 to the rest of the at least two culture medium reservoirs 202, 204; 502, 504 in each cell culture module 106 of the one or more cell culture modules for a first predefined flow time period, the first predefined flow time period being selected based on the first cell culture medium; and
substep S802(d): incubating the cell culture apparatus 100 for a first predefined incubating time period, thereby forming a cell culture comprising a plurality of cells of a first type of cells in each second chamber 208, 508 of each cell culture module 106 of the one or more cell culture modules of the cell culture apparatus 100, the first predefined incubating time period being selected based on the first type of cells. The causing the first cell culture medium to flow may be caused by a flow driving unit 104.

Alternatively, method 800 comprises steps S802 and S804, wherein step S802 comprises substeps S802b(a)-S802b(f):
substep S802b(a): providing a cell culture apparatus 100;
substep S802b(b): providing a fifth cell culture medium via the first chamber 206, 506, or via the first compartment 251 or the second compartment 252 of the first chamber 206, of each cell culture module 106 of the one or more cell culture modules 106 towards the first chamber side 231, 521 of the wall 214, 514, wherein the fifth cell culture medium comprises a fifth type of cells;
substep S802b(c): incubating the cell culture apparatus 100 for a fifth predefined incubating time period, thereby forming a cell culture comprising a plurality of cells of a fifth type of cells in each first chamber 206, 506 or in each first 251 or second compartment 252 of each cell culture module 106 of the one or more cell culture modules of the cell culture apparatus 100, the fifth predefined incubating time period being selected based on the fifth type of cells;
substep S802b(d): providing a first cell culture medium to one of the at least two medium reservoirs 202, 204; 502, 504 of each cell culture module of the one or more cell culture modules 106, wherein the first cell culture medium comprises a first type of cells;
substep S802b(e): causing the first cell culture medium to flow from said one of the at least two culture medium reservoirs 202, 204; 502, 504 via the second chamber 208, 508 to the rest of the at least two culture medium reservoirs 202, 204; 502, 504 in each cell culture module 106 of the one or more cell culture modules for a first predefined flow time period, the first predefined flow time period being selected based on the first cell culture medium; and
substep S802b(f): incubating the cell culture apparatus 100 for a first predefined incubating time period, thereby forming a cell culture comprising a plurality of cells of a first type of cells in each second chamber 208, 508 of each cell culture module 106 of the one or more cell culture modules of the cell culture apparatus 100, the first predefined incubating time period being selected based on the first type of cells; wherein step S804 comprises:
   applying one or more first force
      i) on one or more first locations on the bottom side 230, 530 of the bottom wall 209, 509 of the second chamber 208, 508 such that the bottom wall 209, 509 of the second chamber 208, 508 deforms in the third direction,
   thereby removing at least one cell from the plurality of cells of the first type of cells of the cell culture and forming a first affected cell culture. It is to be understood that applying one or more (first) force may additionally, or alternatively, to removing at least one cell, damage at least one cell. The causing the first cell culture medium to flow may be caused by a flow driving unit 104.

Additionally, or alternatively, the method 800 may further comprise, after the step S804, a step S806 comprising substeps S806(c)-S806(e):
substep S806(c): providing a second cell culture medium comprising a second type of cells to one of the at least two medium reservoirs 202, 204; 502, 504 of each cell culture module 106 of the one or more cell culture modules;
substep S806(d): causing the second culture medium to flow from said one of the at least two culture medium reservoirs 202, 204; 502, 504 via the second chamber 208, 508 to the rest of the at least two culture medium reservoirs 202, 204; 502, 504 in each cell culture module 106 of the one or more cell culture modules for a second predefined flow time period, the second predefined flow time period being selected based on the second culture medium; and
substep S806(e): incubating the cell culture apparatus 100 for a second predefined incubating time period thereby forming a first cultured affected cell culture, the second predefined incubating time period being selected based on the first and second type of cells. In substep S806(d) causing the second cell culture medium to flow may be caused by a flow driving unit 104.

Additionally, or alternatively, the method 800 may further comprise, before or after the step S806, a step S808: applying one or more second force
i) on one or more third locations on the bottom side 230 of the bottom wall 209 of the second chamber 208, the one or more third locations being different from the one or more first locations, such that the bottom wall 209 of the second chamber 208 deforms in the third direction; and/or
ii) on one or more fourth locations on the first chamber side 231 of the wall 214, the one or more fourth locations being different from the one or more second locations, such that the wall deforms in the second direction,
thereby removing at least one cell from the first affected cell culture (of step S804), or from the first cultured affected cell culture (of step S806), and forming a second affected cell culture or a second affected cultured cell culture. Step S808 may further comprise substeps S808(b)-S808(d):
substep S808(b): providing a third cell culture medium comprising a third type of cells to one of the at least two medium reservoirs 202, 204; 502, 504 of each cell culture module 106 of the one or more cell culture modules;
substep S808(c): causing the third culture medium to flow from said one of the at least two culture medium reservoirs 202, 204; 502, 504 via the second chamber 208 to the rest of the at least two culture medium reservoirs 202, 204; 502, 504 in each cell culture module 106 of the one or more cell culture modules for a third predefined flow time period, the third predefined flow time period being selected based on the third culture medium; and
substep S808(d): incubating the cell culture apparatus 100 for a third predefined incubating time period, the third predefined incubating time period being selected based on the first type of cells and/or the third type of cells.

Additionally, or alternatively, one or more of the causing the first (in embodiments of the method 800 comprising causing the first culture medium to flow in in substep S802(c)) and/or second (substep S806(d)) and/or third culture medium to flow (in embodiments of the method 800 comprising causing the third culture medium to flow in substep S806(d)) from the one of the at least two culture medium reservoirs 202, 204; 502, 504 via the second chamber 208 to the rest of the at least two culture medium reservoirs 202, 204; 502, 504 in each cell culture module 106 of the one or more cell culture modules comprises applying a positive pressure to the at least two culture medium reservoirs 202, 204; 502, 504 in each cell culture module 106 of the one or more cell culture modules for the first (in embodiments of the method 800 comprising causing the first culture medium to flow in in substep S802(c)) and/or second (substep S806(d)) and/or third (in embodiments of the method 800 comprising causing the third culture medium to flow in substep S808(c)) predefined flow time period (e.g., 60 min for each first, second, and third predefined flow time period).

In another embodiments, at least one of substep S802(c), substep S806(d), and substep S806(d) of the method 800 may be performed by: (i) applying a first positive pressure to the first culture medium reservoir 202, 502 in each cell culture module 106 (of the cell culture plate 102) for the first and/or second and/or third predefined time interval, while capping the second culture medium reservoir 204, 504; and (ii) applying a second positive pressure to the second culture medium reservoir 204, 504 in each cell culture module 106 (of the cell culture plate 102) for the first and/or second and/or third predefined time interval, while capping the first culture medium reservoir 202, 502. The selection of each of the above-indicated embodiments of the substep S802(c), substep S806(d), and/or substep S806(d) depends on particular applications.

Additionally, or alternatively, the method 800 may further comprise, after step S802 and before or after the step S804, before or after step S806, or before or after step S808, a step S810 comprising substeps S810(a)- S810(b):
S810(a): providing a fourth cell culture medium comprising a fourth type of cells via the first chamber 206, 506 of each cell culture module 106 of the one or more cell culture modules towards the first chamber side 231 of the wall 214; and
S810(b): incubating the cell culture apparatus 100 for a fourth predefined incubating time period, the fourth predefined incubating time period being selected based on the fourth type of cells. In these embodiments, the wall 214 of each cell culture module 106 of the one or more cell culture modules comprises one or more through pores 240 formed therein and the wall is arranged between the first chamber 206, 506 and the second chamber 208, 508 such that the first chamber 206, 506 and the second chamber 208, 508 are in flow communication with each other via the one or more through pores 240. Furthermore, any of the applying one or more force(s) (in step S804 and in the optional step S808) is on the one or more first and/or third locations on the bottom side 230, 530 of the bottom wall 209, 509 of the second chamber 208, 508.

Additionally, or alternatively, the method 800 may further comprise, after step S802 and before or after the step S804, before or after step S806, or before or after step S808, a step S812 comprising substeps S812(a)-S812(c):
substep S812(a): providing a fourth cell culture medium comprising a fourth type of cells via a first compartment 251 of the first chamber 206 of each cell culture module 106 of the one or more cell culture modules towards the first chamber side 231 of the wall 214;
substep S812(c): providing a fifth cell culture medium comprising a fifth type of cells via a second compartment 252 of the first chamber 206 of each cell culture module 106 of the one or more cell culture modules towards the first chamber side 231 of the wall 214; and
substep S812(c): incubating the cell culture apparatus for a fifth predefined incubating time period, the fifth predefined incubating time period being selected based on the fourth and fifth type of cells. In these embodiments, the wall 214 of each cell culture module 106 of the one or more cell culture modules comprises one or more through pores 240 formed therein and the wall being arranged between the first chamber 206 and the second chamber 208 such that the first chamber 206 and the second chamber 208 are in flow communication with each other via the one or more through pores 240. Furthermore, the first chamber comprises the at least one compartment wall 260, and wherein the at least one wall being arranged between the first compartment 251 and the second compartment 252 such that the first compartment 251 and the second compartment 252 are in flow communication with each other. Furthermore, any of the applying one or more force(s) (in step S804 and in the optional step S808) is on the one or more first and/or third locations on the bottom side 230, 530 of the bottom wall 209 of the second chamber 208.

Additionally, or alternatively, the method 800 may further comprise, after the causing the first culture medium to flow (substep 5802(c)) and/or the causing the second culture medium to flow (substep S806(d)) and/or causing the third culture medium to flow (substep S808(c)), or any combinations thereof, a step S814 comprising substeps S814(a)-S814(b), wherein the step S814 comprising substeps S814(a)-S814(b) replaces substep S802(d) and/or substep S806(e) and/or substep S808(d), or any combinations thereof:
substep S814(a): placing the cell culture apparatus 100 in an inverted position; and
substep S814(b): incubating the cell culture apparatus 100 in the inverted position for a sixth predefined incubating time period, the sixth predefined incubating time period being selected based on the culture medium present in the second chamber. Therefore, it is to be understood that two successive substeps of incubating the cell culture apparatus 100 may not be performed. E.g., if substep S802(c) is followed by the step S814 comprising substeps S814(a)-S814(b), substep S802(d) is not performed. Furthermore, if step S814 is performed and the method S800 continues after step S814 (with, e.g., step S804, step S810, or step S812), step S814 may further comprise substep S814(c): placing the cell culture apparatus in an upright position.

Any of the predefined flow time periods (e.g., the first, the second, and the third predefined flow time period) may be selected based on the type(s) of cells (e.g., the first, the second, and the third type of cells) comprised in the cell culture medium (e.g., the first, the second, and the third cell culture medium). In particular, the cell deposition in the second chamber 208, 508, in particular on the upper side 232 of the bottom wall 209, 509 of the second chamber 208, 508, on the basal surface 233, 533 of the wall 214, 514 (comprising or being free of one or more through pores formed therein), or on the one or more biological material growing means 270 comprised in the second chamber 208, 508 may be achieved if a flow rate is greater than gravity and a continuous flow lasts at least 30 min, preferably 60 min.

If the first chamber 206, 506 is used for providing a (fourth and/or fifth) cell culture medium comprising a fourth and/or fifth type of cells via the first chamber 206, 506 (or via the first and/or second compartment 252 of the first chamber 206) towards the first chamber side 231, 531 of the wall 214, 514, a cell (mono)layer, spheroids, and/or organoids may be formed on the first chamber side 231, 531 (i.e., on the apical surface) of the wall 214, 514 and this cell (mono)layer may interact, via the through pores of the wall 214, 514, with a cell layer forming in the second chamber 208, 508, in particular on the upper side 232 of the bottom wall 209, 509 of the second chamber 208, 508, on the basal surface 233, 533 of the wall 214, 514 (comprising one or more through pores formed therein), or on the one or more biological material growing means 270 comprised in the second chamber 208, 508. In particular, if the first chamber 206 comprising at least two compartments and at least one compartment wall 260 is used, the first 251 and second compartment 252 may comprise cells, spheroids, and/or organoids from the fourth and/or fifth cell culture medium that are trapped in said first 251 and second compartments 251 that may form a single spheroid and/or organoid in each compartment. These single spheroids and/or organoids may interact with each other due to the first 251 and second compartment 252 being in flow communication with each other. In this case, the flow channels 510 and 512 may perfuse cell culture medium through the wall 514 via the one or more through pores formed therein to the first 251 and/or second compartment 251 to the keep the spheroids and/or organoids alive. Alternatively, if the first chamber 506 is used, multiple microcavities 524 are bonded to the wall 514 comprising one or more through pores formed therein, for which reason cells from the fourth and/or fifth cell culture medium are trapped to form a single spheroid and/or organoid in each cavity. In this case, the flow channels 510 and 512 may perfuse cell culture medium through the wall 514 to keep the spheroids and/or organoids alive.

Additionally, or alternatively, the first type of cells in substep S802(b), the second type of cells in substep S806(c), the third type of cells in substep S808(b), the fourth type of cells in substep S810(a) or substep S812(a), and the fifth type of cells in substep S812(c) may be each independently selected from endothelial cells, epithelial cells, musculoskeletal cells or induced pluripotent stem cells (IPSC) derived cells or -differentiated cells. These first, second, third, fourth, and fifth type of cells may be in a form of a monolayer, hydrogel embedded cell suspension, monolayer, or thick layered cell layer. Examples of first, second, third, fourth, and fifth type of cells include, but are not limited to, vascular endothelial cells, such as human vascular endothelial cells, or muscular cells.

Additionally, or alternatively, the first, second, third, fourth, and fifth type of cells include, but are not limited to, vascular endothelial cells, such as human vascular endothelial cells; astrocytes, such as human or mouse astrocytes; or muscular cells.

The culture medium perfused through the flow channels 210 (510) and 212 (512) may comprise human brain vascular endothelial cells, while the different culture medium fed to the first chamber 206 (505) may comprise human astrocytes. By using these types of cells, it is possible to mimic an artificial blood-brain barrier (BBB).

### Experimental results

The apparatus 100 comprising the cell culture modules like the one shown in FIGs. 2A-2C was used in accordance with the method 800 to obtain BBB models based on mouse astrocytes and human brain endothelial cells. Each BBB model was obtained in two stages which are described below in more detail.

### I. Stage of preparing the apparatus 100:

### Materials:

| | |
|---|---|
| Extracellular Matrix (ECM) coating solution (used as a cell adhesion enhancing layer for the flow channels 210, 212, the second chamber 208, and the first chamber 206) | Collagen I (Col-I) 100 µg/mL, 0.1% acetic acid |
| Washing | 1xPBS (phosphate buffer saline), ethanol 96% |
| Plate 102 | 96-well format |

The stage of preparing was performed as follows:
1. The dry apparatus 100 was treated with plasma for 60 s.
2. The apparatus 100 was washed with 96% ethanol. 50 µL of 96 % ethanol was added to each open-top (first) chamber 206.
3. The ethanol inside the flow channels 210, 212 and the first chamber 206 was replaced/washed twice with 50 µL of PBS.
4. 50 µL of Col-I coating solution was injected into the flow channels 210, 212.
5. 50 µL of Col-I coating solution was added into each first chamber 206 of the apparatus 100.
6. Each culture medium reservoir was filled with 50 µL PBS, closed, and incubated at 37°C, 5 % CO₂, for 1 hour on the rocking platform (1 cycle per 15 min).
7. After incubation, the coating solution in the flow channels 210, 212 and each first chamber 206 of the apparatus 100 was replaced with PBS.

### II. Stage of obtaining the BBB model:

### Materials:

| | |
|---|---|
| Cells used for the BBB model (concentration, volume) | Mouse astrocytes (MA) (2 × 10⁶ cells/mL, min. 4.8 mL), mouse primary brain microvascular endothelial cells (MPBMEC) (2 × 10⁶ cells/mL, min. 4.8 mL). |
| | Human astrocytes (HA) (2 × 10⁶ cells/mL, min. 4.8mL), human primary brain microvascular endothelial cells (HPBMEC) (2 × 10⁶ cells/mL, min. 4.8mL). |

The BBB model based on the mouse cells (i.e., MA-cells and MPBMEC) was obtained as follows:
1. 4800 µL MA-cells at 2 × 10⁶ cells/mL were prepared according to the conventional cell culture protocols.
2. The MA-cells were placed on ice.
3. The MA-cell loading was performed by:
   a. removing all the PBS from the first chambers 206,
   b. replacing the PBS in the flow channels 210, 212 with µL 100 endothelial cell medium,
   c. pipetting 50 µL MA-cell suspension at 2 × 10⁶ cells/mL into the first chambers 206,
   d. incubating the apparatus 100 at 37°C, 5 % CO₂, for 1 hour (static, not on the rocking platform),
   e. checking every 10 min that the wall 214 does not dry out (if required, a fresh culture medium may be added to the first chamber(s) 206 to keep the wall 214 moist).
4. 4800 µL MPBMEC at 2 × 10⁶ cells/mL were prepared according to the conventional cell culture protocols.
5. The MPBMEC were again placed on ice.
6. The cell culture medium (added in the apparatus in step 3e) was replaced by injecting 50 µL MPBMEC suspension at 2 × 10⁶ cells/mL into the flow channels 210, 212 of the plate 102.
7. The plate 102 was manually rocked a few times to make sure the cell confluency is consistent.
8. The plate 102 was inverted and incubated at 37°C, 5 % CO₂, for 1 hour (static, not on the rocking platform).
9. 100 µL MPBMEC were added to the culture medium reservoir at one side of each flow channel in the plate 102.
10. 15 µL MPBMEC were added to the culture medium reservoir at one side of each flow channel in the plate 102.
11. The plate 102 was placed on the rocking platform at 37°C, 5 % CO₂.

The BBB model based on the human cells (i.e., HA-cells and HPBMEC) may be obtained in the same manner (one just needs to replace "MA" and "MPBMEC" with "HA" and "HPBMEC", respectively, in steps 1-11 above).

Thus, the MAs were introduced in the first chamber 206 and grown a monolayer on the apical surface of the wall 214, while the MPBMEC were perfused through the second chamber 208 and deposited on the basal side of the wall 214.

Subsequently, the cells were treated immunohistochemically with antibodies against CD31, glial fibrillary acidic protein (GFAP), Actin and DAPI (which is a small molecule that binds to DNA showing the cell nuclei).

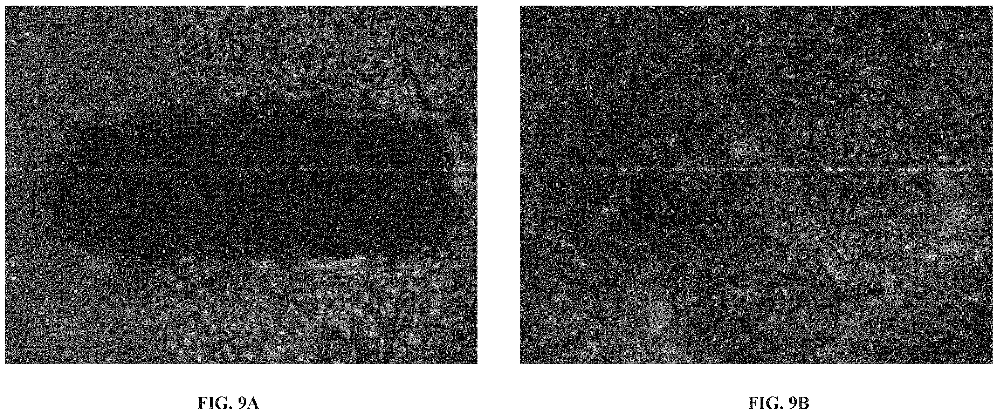

FIGs. 9A and 9B show confocal images of the BBB model by using the apparatus 100. More specifically, FIG. 9A shows the basal surface of the wall 214, on which the MPBMEC were cultured. Adherens junction marker CD31 (used as the cell type-specific marker of the MPBMEC) and actin expression have shown robust expression and indicated intact cell junctions. FIG. 9B shows the apical surface of the wall 214 (from the side of the first chamber 206), on which the MAs were cultured. FIG. 9B shows the GFAP (used as the cell type-specific marker of the mouse brain astrocytes) and actin expression.

## Claims

1. A cell culture apparatus (100) comprising:
one or more cell culture modules (106, 400), each cell culture module of the one or more cell culture modules comprising:
- at least two culture medium reservoirs (202, 204; 502, 504) each having a top part and a bottom part, the top part having an inlet (216, 220; 516, 520) for a culture medium and the bottom part having an outlet (218, 222; 518, 522) for the culture medium;
- a first chamber (206, 506) arranged between the at least two culture medium reservoirs (202, 204; 502, 504) such that the first chamber and the at least two culture medium reservoirs are aligned with each other in a first direction;
- a second chamber (208, 508) arranged under the first chamber (206, 506) and aligned with the first chamber in a second direction, the second direction having an angle being more than 0 degrees but 90 degrees or less in respect to the first direction, the second chamber having a bottom part having at least two lateral holes;
- a wall (214, 514) arranged between the first chamber (206, 506) and the second chamber (208, 508); and
- at least two flow channels (210, 212; 510, 512), each of the at least two flow channels being configured to pass a cell culture medium therethrough, and each of the at least two flow channels (210, 212; 510, 512) connecting the outlet (218, 222; 518, 522) of the bottom part of one of the at least two culture medium reservoirs (202, 204; 502, 504) to one of the at least two lateral holes of the bottom part of the second chamber (208, 508),
wherein the second chamber has a bottom wall (209, 509) made of an elastic bottom wall material configured to deform in a third direction, the third direction being opposite to the second direction, in response to a force applied on the bottom side (230, 530) of the bottom wall of the second chamber and/or the wall (214) is made of an elastic wall material configured to deform in the second direction in response to a force applied on the first chamber side (231, 531) of the wall (214, 514).

2. The cell culture apparatus as claimed in claim 1, wherein the apparatus comprises a plurality of cell culture modules arranged adjacent to each other.

3. The cell culture apparatus as claimed in any of the preceding claims, wherein the cell culture module further comprises one or more protrusions (280) attached to and protruding from the wall on the apical side (231, 531) or the basal side (233, 533) of the wall, or attached to and protruding from the upper side (232, 532) of the bottom wall of the second chamber.

4. The cell culture apparatus as claimed in any of the preceding claims, wherein the second chamber further comprises one or more biological material growing means.

5. The cell culture apparatus as claimed in any of the preceding claims, wherein the wall comprises one or more through pores (240) formed therein and the wall being arranged between the first chamber and the second chamber such that the first chamber and the second chamber are in flow communication with each other via the one or more through pores.

6. The cell culture apparatus as claimed in any of claims 1 - 5, wherein the bottom part of the second chamber is made of the elastic bottom part material configured to deform in the third direction, the third direction being opposite to the second direction, in response to a force applied on the bottom side of the bottom wall of the second chamber; the first chamber comprises at least two compartments and at least one compartment wall (260), wherein the at least two compartments comprise a first compartment (251) and a second compartment (252), wherein the at least one compartment wall being arranged between the first compartment and the second compartment such that the first compartment and the second compartment are in flow communication with each other; and the wall (214) comprises one or more through pores formed therein and the wall (214) being arranged between the first chamber and the second chamber such that the first chamber and the second chamber are in flow communication with each other via the one or more through pores.

7. The cell culture apparatus as claimed in any of claims 1 - 6, wherein the wall material of the wall is an elastic wall material configured to deform in the second direction in response to a force applied on the first chamber side of the wall.

8. The cell culture apparatus as claimed in any of claims 1 - 7, wherein the bottom wall of the second chamber is made of an elastic bottom wall material configured to deform in a third direction, the third direction being opposite to the second direction, in response to a force applied on the bottom side of the bottom wall of the second chamber.

9. The cell culture apparatus as claimed in any of claims 1 - 8, wherein the second chamber of at least one cell culture module of the one or more cell culture modules comprises at least one cell adhesion enhancing layer being made of a hydrophilic material, the at least one cell adhesion enhancing layer being provided on an inner surface of the second chamber, provided that if the wall comprises one or more through pores formed therein the at least one cell adhesion enhancing layer being provided on the inner surface of the second chamber such that the one or more through pores of the wall remain open.

10. The cell culture apparatus as claimed in any of claims 1 - 9, wherein the wall of at least one cell culture module of the one or more cell culture modules comprises at least one cell adhesion enhancing layer being made of a hydrophilic material, the at least one cell adhesion enhancing layer being provided on the wall, provided that if the wall comprises one or more through pores formed therein the at least one cell adhesion enhancing layer being provided on the wall such that the one or more through pores of the wall remain open, wherein the hydrophilic material of the at least one cell adhesion enhancing layer is selected based on at least one type of cells to be grown on the wall.

11. The cell culture apparatus as claimed in any of claims 1 - 10, wherein the first chamber in at least one cell culture module of the one or more cell culture modules is implemented as a bottomless hollow tube.

12. The cell culture apparatus as claimed in any of claims 1 - 11, wherein the first chamber in at least one cell culture module of the one or more cell culture modules is implemented as a hollow tube having a curved or angled bottom, the curved or angled bottom having at least one first chamber cavity (524) and at least one first chamber hole formed in each of the at least one first chamber cavity, and wherein the bottom wall of the second chamber (508) is made of an elastic bottom wall material configured to deform in a third direction, the third direction being opposite to the second direction, in response to a force applied on the bottom side of the bottom wall of the second chamber.

13. The cell culture apparatus of claim 12, wherein the curved or angled bottom is coated, from outside, with a cell adhesion enhancing layer such that the at least one first chamber hole of the at least one first chamber cavity remains open, the cell adhesion enhancing layer being made of a hydrophilic material.

14. The cell culture apparatus as claimed in any of claims 1 - 13, wherein the second chamber of at least one cell culture module of the one or more cell culture modules has a height falling within a range selected from 50 - 350 µm.

15. The cell culture apparatus as claimed in any of claims 1 - 14, wherein each of the at least two culture medium reservoirs in at least one cell culture module of the one or more cell culture modules is implemented as a hollow tube.

16. The cell culture apparatus as claimed in any of claims 1 - 15, wherein at least one cell culture module of the one or more cell culture modules further comprises at least two electrodes, wherein each of the at least two electrodes is independently arranged in the first chamber, the second chamber, in one or more of the at least two culture medium reservoirs, or the wall, or any combinations thereof.

17. The cell culture apparatus as claimed in any of claims 1 - 16, wherein the one or more through pores of the wall has an average pore size falling within a range from 0.2 µm to 200 µm.

18. A cell culture incubator comprising:
a cell culture apparatus (100) as defined in any of claims 1 - 17; and
a pipetting station (702) configured to feed the culture medium to each of the at least two culture medium reservoirs in each cell culture module of the one or more cell culture modules.

19. A method for affecting a cell culture, the method comprising:
(a) providing a cell culture apparatus as defined in any of claims 1 - 17, wherein each second chamber of each cell culture module of the one or more cell culture modules comprises a cell culture comprising a plurality of cells of a first type of cells;
(b) applying one or more first force
i) on one or more first locations on the bottom side of the bottom wall of the second chamber such that the bottom wall of the second chamber deforms in the third direction; and/or
ii) on one or more second locations on the first chamber side of the wall such that the wall deforms in the second direction,
thereby removing at least one cell from the plurality of cells of the first type of cells of the cell culture and forming a first affected cell culture.

20. The method as claimed in claim 19, wherein the method further comprises:
(c) providing a second cell culture medium comprising a second type of cells to one of the at least two medium reservoirs of each cell culture module of the one or more cell culture modules;
(d) causing the second culture medium to flow from said one of the at least two culture medium reservoirs via the second chamber to the rest of the at least two culture medium reservoirs in each cell culture module of the one or more cell culture modules for a second predefined flow time period, the second predefined flow time period being selected based on the second culture medium; and
(e) incubating the cell culture apparatus for a second predefined incubating time period, thereby forming a first cultured affected cell culture, the second predefined incubating time period being selected based on the first and second type of cells.

21. The method as claimed claim 20, wherein, in (d), the causing the second cell culture medium to flow is caused by a flow driving unit.

22. The method as claimed in any of claims 19 - 21, wherein the second type of cells being same as, or different to, the first type of cells, and, in (e), the second predefined incubating time period being selected based on the first and second type of cells.

23. The method as claimed in any of claims 19 - 22, wherein the method further comprises:
- applying one or more second force
i) on one or more third locations on the bottom side of the bottom wall of the second chamber, the one or more third locations being different from the one or more first locations, such that the bottom wall of the second chamber deforms in the third direction; and/or
ii) on one or more fourth locations on the first chamber side of the wall, the one or more fourth locations being different from the one or more second locations, such that the wall deforms in the second direction,
thereby removing at least one cell from the first affected cell culture, or from the first cultured affected cell culture, and forming a second affected cell culture or a second affected cultured cell culture;
- providing a third cell culture medium comprising a third type of cells, to one of the at least two medium reservoirs of each cell culture module of the one or more cell culture modules;
- causing the third culture medium to flow from said one of the at least two culture medium reservoirs via the second chamber to the rest of the at least two culture medium reservoirs in each cell culture module of the one or more cell culture modules for a third predefined flow time period, the third predefined flow time period being selected based on the third culture medium; and
- incubating the cell culture apparatus for a third predefined incubating time period, the third predefined incubating time period being selected based on the first type of cells and/or the third type of cells.

24. The method as claimed in any of claims 19 - 23, wherein the method further comprises:
- providing a fourth cell culture medium comprising a fourth type of cells via the first chamber of each cell culture module of the one or more cell culture modules towards the first chamber side of the wall; and
- incubating the cell culture apparatus for a fourth predefined incubating time period, the fourth predefined incubating time period being selected based on the fourth type of cells,
wherein the wall of each cell culture module of the one or more cell culture modules comprises one or more through pores formed therein and the wall being arranged between the first chamber and the second chamber such that the first chamber and the second chamber are in flow communication with each other via the one or more through pores, and wherein any of the applying one or more force(s) is on the one or more first and/or third locations on the bottom side of the bottom wall of the second chamber.

25. The method as claimed in any of claims 19 - 24, wherein the method further comprises:
- providing a fourth cell culture medium comprising a fourth type of cells via a first compartment of the first chamber of each cell culture module of the one or more cell culture modules towards the first chamber side of the wall;
- providing a fifth cell culture medium comprising a fifth type of cells via a second compartment of the first chamber of each cell culture module of the one or more cell culture modules to the first chamber side of the wall; and
- incubating the cell culture apparatus for a fifth predefined incubating time period, the fifth predefined incubating time period being selected based on the fourth and fifth type of cells,
wherein the wall of each cell culture module of the one or more cell culture modules comprises one or more through pores formed therein and the wall being arranged between the first chamber and the second chamber such that the first chamber and the second chamber are in flow communication with each other via the one or more through pores,
wherein the first chamber comprises the at least one compartment wall, and wherein the at least one wall being arranged between the first compartment and the second compartment such that the first compartment and the second compartment are in flow communication with each other, and wherein any of the applying one or more force(s) is on the one or more first and/or third locations on the bottom side of the bottom wall of the second chamber.

26. The method as claimed in any of claims 19 - 25, wherein the first, second, third, fourth, and/or fifth type of cells is each independently selected from the group consisting of astrocytes, brain organoids, brain spheroids, neuronal cells, endothelial cells, epithelial cells, musculoskeletal cells, induced pluripotent stem cells (IPSC) derived or differentiated cells, and muscular cells.

## Patentansprüche

1. Zellkulturvorrichtung (100), umfassend:
ein oder mehrere Zellkulturmodule (106, 400), wobei jedes Zellkulturmodul des einen oder der mehreren Zellkulturmodule Folgendes umfasst:
- mindestens zwei Kulturmediumreservoirs (202, 204; 502, 504) mit jeweils einem Oberteil und einem Bodenteil, wobei das Oberteil einen Einlass (216, 220; 516, 520) für ein Kulturmedium aufweist und das Bodenteil einen Auslass (218, 222; 518, 522) für das Kulturmedium aufweist;
- eine erste Kammer (206, 506), die zwischen den mindestens zwei Kulturmediumreservoirs (202, 204; 502, 504) so angeordnet ist, dass die erste Kammer und die mindestens zwei Kulturmediumreservoirs in einer ersten Richtung miteinander ausgerichtet sind;
- eine zweite Kammer (208, 508), die unterhalb der ersten Kammer (206, 506) angeordnet und mit der ersten Kammer in einer zweiten Richtung ausgerichtet ist, wobei die zweite Richtung einen Winkel von mehr als 0 Grad, aber 90 Grad oder weniger in Bezug zur ersten Richtung aufweist, wobei die zweite Kammer ein Bodenteil aufweist, das mindestens zwei laterale Löcher aufweist;
- eine Wand (214, 514) die zwischen der ersten Kammer (206, 506) und der zweiten Kammer (208, 508) angeordnet ist; und
- mindestens zwei Strömungskanäle (210, 212; 510, 512), wobei jeder der mindestens zwei Strömungskanäle dazu konfiguriert ist, ein Zellkulturmedium durch ihn hindurchfließen zu lassen, und wobei jeder der mindestens zwei Strömungskanäle (210, 212; 510, 512) den Auslass (218, 222; 518, 522) des Bodenteils eines der mindestens zwei Kulturmediumreservoirs (202, 204; 502, 504) mit einem der mindestens zwei lateralen Löcher des Bodenteils der zweiten Kammer (208, 508) verbindet, wobei die zweite Kammer eine Bodenwand (209, 509) aufweist, die aus einem elastischen Bodenwandmaterial hergestellt ist, das dazu konfiguriert ist, sich in einer dritten Richtung zu deformieren, wobei die dritte Richtung entgegengesetzt zur zweiten Richtung ist, in Antwort darauf, dass eine Kraft auf die Bodenseite (230, 530) der Bodenwand der zweiten Kammer ausgeübt wird und/oder wobei die Wand (214) aus einem elastischen Wandmaterial hergestellt ist, das dazu konfiguriert ist, sich in der zweiten Richtung zu deformieren, in Antwort darauf, dass eine Kraft auf die erste Kammerseite (231, 531) der Wand (214, 514) ausgeübt wird.

2. Zellkulturvorrichtung nach Anspruch 1, wobei die Vorrichtung eine Mehrzahl von Zellkulturmodulen umfasst, die benachbart zueinander angeordnet sind.

3. Zellkulturvorrichtung nach einem der vorhergehenden Ansprüche, wobei das Zellkulturmodul ferner einen oder mehrere Vorsprünge (280) umfasst, die an der Wand auf der apikalen Seite (231, 531) oder der basalen Seite (233, 533) der Wand angebracht sind und von dieser hervorstehen, oder an der oberen Seite (232, 532) der Bodenwand der zweiten Kammer angebracht sind und von dieser hervorstehen.

4. Zellkulturvorrichtung nach einem der vorhergehenden Ansprüche, wobei die zweite Kammer ferner eine oder mehrere Mittel zum Wachsen von biologischem Material umfasst.

5. Zellkulturvorrichtung nach einem der vorhergehenden Ansprüche, wobei die Wand eine oder mehrere darin gebildete Durchgangsporen (240) umfasst und die Wand zwischen der ersten Kammer und der zweiten Kammer so angeordnet ist, dass die erste Kammer und die zweite Kammer über die eine oder mehrere Durchgangsporen in Strömungsverbindung miteinander sind.

6. Zellkulturvorrichtung nach einem der Ansprüche 1 bis 5, wobei das Bodenteil der zweiten Kammer aus dem elastischen Bodenteilmaterial hergestellt ist, das dazu konfiguriert ist, sich in der dritten Richtung zu deformieren, wobei die dritte Richtung der zweiten Richtung entgegengesetzt ist, in Antwort darauf, dass eine Kraft auf die Bodenseite der Bodenwand der zweiten Kammer ausgeübt wird; wobei die erste Kammer mindestens zwei Abteile und mindestens eine Abteilwand (260) umfasst, wobei die mindestens zwei Abteile ein erstes Abteil (251) und ein zweites Abteil (252) umfassen, wobei die mindestens eine Abteilwand zwischen dem ersten Abteil und dem zweiten Abteil so angeordnet ist, dass das erste Abteil und das zweite Abteil in Strömungsverbindung miteinander sind; und die Wand (214) eine oder mehrere darin gebildete Durchgangsporen umfasst und die Wand (214) zwischen der ersten Kammer und der zweiten Kammer so angeordnet ist, dass die erste Kammer und die zweite Kammer über die eine oder mehrere Durchgangsporen in Strömungsverbindung miteinander sind.

7. Zellkulturvorrichtung nach einem der Ansprüche 1 - 6, wobei das Wandmaterial der Wand ein elastisches Wandmaterial ist, das dazu konfiguriert ist, sich in der zweiten Richtung zu deformieren, in Antwort darauf, dass eine Kraft auf die erste Kammerseite der Wand ausgeübt wird.

8. Zellkulturvorrichtung nach einem der Ansprüche 1 - 7, wobei die Bodenwand der zweiten Kammer aus einem elastischen Bodenwandmaterial hergestellt ist, das dazu konfiguriert ist, sich in einer dritten Richtung zu deformieren, wobei die dritte Richtung der zweiten Richtung entgegengesetzt ist, in Antwort darauf, dass eine Kraft auf die Bodenseite der Bodenwand der zweiten Kammer ausgeübt wird.

9. Zellkulturvorrichtung nach einem der Ansprüche 1 - 8, wobei die zweite Kammer mindestens eines Zellkulturmoduls des einen oder der mehreren Zellkulturmodule mindestens eine Zelladhäsionsverstärkungsschicht, die aus einem hydrophilen Material hergestellt ist, umfasst, wobei die mindestens eine Zelladhäsionsverstärkungsschicht auf einer inneren Oberfläche der zweiten Kammer bereitgestellt ist, vorausgesetzt, dass, wenn die Wand eine oder mehrere darin gebildete Durchgangsporen umfasst, die mindestens eine Zelladhäsionsverstärkungsschicht auf der inneren Oberfläche der zweiten Kammer so bereitgestellt ist, dass die eine oder mehrere Durchgangsporen der Wand offen bleiben.

10. Zellkulturvorrichtung nach einem der Ansprüche 1 - 9, wobei die Wand mindestens eines Zellkulturmoduls des einen oder der mehreren Zellkulturmodule mindestens eine Zelladhäsionsverstärkungsschicht, die aus einem hydrophilen Material hergestellt ist, umfasst, wobei die mindestens eine Zelladhäsionsverstärkungsschicht auf der Wand bereitgestellt ist, vorausgesetzt, dass, wenn die Wand eine oder mehrere darin gebildete Durchgangsporen umfasst, die mindestens eine Zelladhäsionsverstärkungsschicht auf der Wand so bereitgestellt ist, dass die eine oder mehrere Durchgangsporen der Wand offen bleiben, wobei das hydrophile Material der mindestens einen Zelladhäsionsverstärkungsschicht basierend auf mindestens einem Zelltyp ausgewählt ist, der auf der Wand wachsen soll.

11. Zellkulturvorrichtung nach einem der Ansprüche 1 - 10, wobei die erste Kammer in mindestens einem Zellkulturmodul des einen oder der mehreren Zellkulturmodule als ein bodenloses hohles Rohr implementiert ist.

12. Zellkulturvorrichtung nach einem der Ansprüche 1 - 11, wobei die erste Kammer in mindestens einem Zellkulturmodul des einen oder der mehreren Zellkulturmodule als ein hohles Rohr mit einem gekrümmten oder abgewinkelten Boden implementiert ist, wobei der gekrümmte oder abgewinkelte Boden mindestens eine erste Kammerkavität (524) und mindestens ein erstes Kammerloch aufweist, das in jedem der mindestens einen ersten Kammerkavität gebildet ist, und wobei die Bodenwand der zweiten Kammer (508) aus einem elastischen Bodenwandmaterial hergestellt ist, das dazu konfiguriert ist, sich in einer dritten Richtung zu deformieren, wobei die dritte Richtung der zweiten Richtung entgegengesetzt ist, in Antwort darauf, dass eine Kraft auf die Bodenseite der Bodenwand der zweiten Kammer ausgeübt wird.

13. Zellkulturvorrichtung nach Anspruch 12, wobei der gekrümmte oder abgewinkelte Boden von außen mit einer Zelladhäsionsverstärkungsschicht beschichtet ist, derart, dass das mindestens eine erste Kammerloch der mindestens einen ersten Kammerkavität offen bleibt, wobei die Zelladhäsionsverstärkungsschicht aus einem hydrophilen Material hergestellt ist.

14. Zellkulturvorrichtung nach einem der Ansprüche 1 - 13, wobei die zweite Kammer mindestens eines Zellkulturmoduls des einen oder der mehreren Zellkulturmodule eine Höhe aufweist, die in einem Bereich liegt, der aus 50 bis 350 µm ausgewählt ist.

15. Zellkulturvorrichtung nach einem der Ansprüche 1 - 14, wobei jedes der mindestens zwei Kulturmediumreservoirs in mindestens einem Zellkulturmodul des einen oder der mehreren Zellkulturmodule als hohles Rohr implementiert ist.

16. Zellkulturvorrichtung nach einem der Ansprüche 1 - 15, wobei mindestens ein Zellkulturmodul des einen oder der mehreren Zellkulturmodule ferner mindestens zwei Elektroden umfasst, wobei jede der mindestens zwei Elektroden unabhängig voneinander in der ersten Kammer, der zweiten Kammer, in einem oder mehreren der mindestens zwei Kulturmediumreservoirs, oder der Wand, oder einer beliebigen Kombination davon angeordnet ist.

17. Zellkulturvorrichtung nach einem der Ansprüche 1 - 16, wobei die eine oder mehrere Durchgangsporen der Wand eine durchschnittliche Porengröße im Bereich von 0,2 µm bis 200 µm aufweisen.

18. Zellkulturinkubator, umfassend:
eine Zellkulturvorrichtung (100) gemäß einem der Ansprüche 1 - 17; und
eine Pipettierstation (702), die dazu konfiguriert ist, das Kulturmedium jedem der mindestens zwei Kulturmediumreservoirs in jedem Zellkulturmodul des einen oder der mehreren Zellkulturmodule zuzuführen.

19. Verfahren zur Beeinträchtigung einer Zellkultur, wobei das Verfahren umfasst:
(a) Bereitstellen einer Zellkulturvorrichtung gemäß einem der Ansprüche 1 - 17, wobei jede zweite Kammer jedes Zellkulturmoduls des einen oder der mehreren Zellkulturmodulen eine Zellkultur umfasst, die eine Mehrzahl von Zellen eines ersten Zelltyps umfasst;
(b) Ausüben einer oder mehrerer erster Kräfte
i) auf eine oder mehrere erste Stellen an der Bodenseite der Bodenwand der zweiten Kammer, derart, dass sich die Bodenwand der zweiten Kammer in der dritten Richtung deformiert; und/oder
ii) auf eine oder mehrere zweite Stellen an der ersten Kammerseite der Wand, derart, dass sich die Wand in der zweiten Richtung deformiert,
wodurch mindestens eine Zelle aus der Mehrzahl von Zellen des ersten Zelltyps der Zellkultur entfernt und eine erste betroffene Zellkultur gebildet wird.

20. Verfahren nach Anspruch 19, wobei das Verfahren ferner Folgendes umfasst:
(c) Bereitstellen eines zweiten Zellkulturmediums, das einen zweiten Zelltyp umfasst, zu einem der mindestens zwei Mediumreservoirs jedes Zellkulturmoduls des einen oder der mehreren Zellkulturmodule;
(d) Verursachen, dass das zweite Kulturmedium aus dem einen der mindestens zwei Kulturmediumreservoirs über die zweite Kammer zu den restlichen der mindestens zwei Kulturmediumreservoirs in jedem Zellkulturmodul des einen oder der mehreren Zellkulturmodule für eine zweite vordefinierte Strömungszeitperiode fließt, wobei die zweite vordefinierte Strömungszeitperiode basierend auf dem zweiten Kulturmedium ausgewählt wird; und
(e) Inkubieren der Zellkulturvorrichtung für eine zweite vordefinierte Inkubationszeitperiode, wodurch eine erste kultivierte betroffene Zellkultur gebildet wird, wobei die zweite vordefinierte Inkubationszeitperiode basierend auf dem ersten und zweiten Zelltyp ausgewählt wird.

21. Verfahren nach Anspruch 20, wobei, in (d), das Verursachen, dass das zweite Zellkulturmedium fließt, durch eine Strömungsantriebseinheit verursacht wird.

22. Verfahren nach einem der Ansprüche 19 - 21, wobei der zweite Zelltyp mit dem ersten Zelltyp übereinstimmt oder von diesem verschieden ist, und, in (e), die zweite vordefinierte Inkubationszeitperiode basierend auf dem ersten und zweiten Zelltyp ausgewählt wird.

23. Verfahren nach einem der Ansprüche 19 - 22, wobei das Verfahren ferner umfasst:
- Ausüben einer oder mehrerer zweiter Kräfte
i) auf eine oder mehrere dritte Stellen an der Bodenseite der Bodenwand der zweiten Kammer, wobei sich die eine oder mehrere dritten Stellen von der einen oder den mehreren ersten Stellen unterscheiden, derart, dass sich die Bodenwand der zweiten Kammer in der dritten Richtung deformiert; und/oder
ii) auf eine oder mehrere vierte Stellen auf der ersten Kammerseite der Wand, wobei sich die eine oder mehreren vierten Stellen von der einen oder den mehreren zweiten Stellen unterscheiden, derart, dass sich die Wand in der zweiten Richtung deformiert,
wodurch mindestens eine Zelle aus der ersten betroffenen Zellkultur, oder aus der ersten kultivierten betroffenen Zellkultur entfernt wird, und eine zweite betroffene Zellkultur oder eine zweite kultivierte betroffene Zellkultur gebildet wird;
- Bereitstellen eines dritten Zellkulturmediums, das einen dritten Zelltyp umfasst, zu einem der mindestens zwei Mediumreservoirs jedes Zellkulturmoduls des einen oder der mehreren Zellkulturmodule;
- Verursachen, dass das dritte Kulturmedium aus dem einen der mindestens zwei Kulturmediumreservoirs über die zweite Kammer zu den restlichen der mindestens zwei Kulturmediumreservoirs in jedem Zellkulturmodul des einen oder der mehreren Zellkulturmodule für eine dritte vordefinierte Strömungszeitperiode fließt, wobei die dritte vordefinierte Strömungszeitperiode basierend auf dem dritten Kulturmedium ausgewählt wird; und
- Inkubieren der Zellkulturvorrichtung für eine dritte vordefinierte Inkubationszeitperiode, wobei die dritte vordefinierte Inkubationszeitperiode basierend auf dem ersten Zelltyp und/oder dem dritten Zelltyp ausgewählt wird.

24. Verfahren nach einem der Ansprüche 19 - 23, wobei das Verfahren ferner umfasst:
- Bereitstellen eines vierten Zellkulturmediums, das einen vierten Zelltyp von Zellen umfasst, über die erste Kammer jedes Zellkulturmoduls des einen oder der mehreren Zellkulturmodule in Richtung der ersten Kammerseite der Wand; und
- Inkubieren der Zellkulturvorrichtung für eine vierte vordefinierte Inkubationszeitperiode, wobei die vierte vordefinierte Inkubationszeitperiode basierend auf dem vierten Zelltyp ausgewählt wird,
wobei die Wand jedes Zellkulturmoduls des einen oder der mehreren Zellkulturmodule eine oder mehrere darin gebildete Durchgangsporen umfasst und die Wand zwischen der ersten Kammer und der zweiten Kammer so angeordnet ist, dass die erste Kammer und die zweite Kammer über die eine oder die mehreren Durchgangsporen in Strömungsverbindung miteinander sind, und wobei jede der einen oder mehreren ausgeübten Kräfte auf die eine oder die mehreren ersten und/oder dritten Stellen auf der Bodenseite der Bodenwand der zweiten Kammer ausgeübt wird.

25. Verfahren nach einem der Ansprüche 19 - 24, wobei das Verfahren ferner umfasst:
- Bereitstellen eines vierten Zellkulturmediums, das einen vierten Zelltyp umfasst, über ein erstes Abteil der ersten Kammer jedes Zellkulturmoduls des einen oder der mehreren Zellkulturmodule in Richtung der ersten Kammerseite der Wand; und
- Bereitstellen eines fünften Zellkulturmediums, das einen fünften Zelltyp umfasst, über ein zweites Abteil der ersten Kammer jedes Zellkulturmoduls des einen oder der mehreren Zellkulturmodule zur ersten Kammerseite der Wand; und
- Inkubieren der Zellkulturvorrichtung für eine fünfte vordefinierte Inkubationszeitperiode, wobei die fünfte vordefinierte Inkubationszeitperiode basierend auf dem vierten und fünften Zelltyp ausgewählt wird,
wobei die Wand jedes Zellkulturmoduls des einen oder der mehreren Zellkulturmodule eine oder mehrere darin gebildete Durchgangsporen umfasst und die Wand zwischen der ersten Kammer und der zweiten Kammer so angeordnet ist, dass die erste Kammer und die zweite Kammer über die eine oder die mehreren Durchgangsporen in Strömungsverbindung miteinander sind,
wobei die erste Kammer die mindestens eine Abteilwand umfasst, und wobei die mindestens eine Wand zwischen dem ersten Abteil und dem zweiten Abteil so angeordnet ist, dass das erste Abteil und das zweite Abteil in Strömungsverbindung miteinander sind, und wobei jede der eine oder mehreren ausgeübten Kräfte auf die eine oder mehreren ersten und/oder dritten Stellen auf der Bodenseite der Bodenwand der zweiten Kammer wirkt.

26. Verfahren nach einem der Ansprüche 19 - 25, wobei der erste, zweite, dritte, vierte und/oder fünfte Zelltyp jeweils unabhängig aus der Gruppe bestehend aus Astrozyten, Hirnorganoiden, Hirnsphäroiden, neuronalen Zellen, Endothelzellen, Epithelzellen, muskuloskelettalen Zellen, induzierten pluripotenten Stammzellen (IPSC) abgeleiteten oder differenzierten Zellen und Muskelzellen ausgewählt ist.

## Revendications

1. Appareil de culture cellulaire (100) comprenant :
un ou plusieurs modules de culture cellulaire (106, 400), chaque module de culture cellulaire du un ou des modules de culture cellulaire comprenant :
- au moins deux réservoirs de milieu de culture (202, 204 ; 502, 504) présentant chacun une partie supérieure et une partie inférieure, la partie supérieure présentant une entrée (216, 220 ; 516, 520) pour un milieu de culture et la partie inférieure présentant une sortie (218, 222 ; 518, 522) pour le milieu de culture ;
- une première chambre (206, 506) agencée entre les au moins deux réservoirs de milieu de culture (202, 204 ; 502, 504) de sorte que la première chambre et les au moins deux réservoirs de milieu de culture sont alignés l'un par rapport à l'autre dans une première direction ;
- une deuxième chambre (208, 508) agencée sous la première chambre (206, 506) et alignée avec la première chambre dans une deuxième direction, la deuxième direction présentant un angle supérieur à 0 degré, mais inférieur ou égal à 90 degrés, par rapport à la première direction, la deuxième chambre présentant une partie inférieure présentant au moins deux trous latéraux ;
- une paroi (214, 514) agencée entre la première chambre (206, 506) et la deuxième chambre (208, 508) ; et
- au moins deux canaux d'écoulement (210, 212 ; 510, 512), chacun des au moins deux canaux d'écoulement étant configuré pour faire passer un milieu de culture cellulaire, et chacun des au moins deux canaux d'écoulement (210, 212 ; 510, 512) reliant la sortie (218, 222 ; 518, 522) de la partie inférieure de l'un des au moins deux réservoirs de milieu de culture (202, 204 ; 502, 504) à l'un des au moins deux trous latéraux de la partie inférieure de la deuxième chambre (208, 508),
dans lequel la deuxième chambre présente une paroi inférieure (209, 509) fabriquée dans un matériau de paroi élastique configuré pour se déformer dans une troisième direction, la troisième direction étant opposée à la deuxième direction, en réponse à une force appliquée sur le côté inférieur (230, 530) de la paroi inférieure de la deuxième chambre et/ou la paroi (214) est fabriquée dans un matériau de paroi élastique configuré pour se déformer dans la deuxième direction en réponse à une force appliquée sur le côté première chambre (231, 531) de la paroi (214, 514).

2. Appareil de culture cellulaire selon la revendication 1, dans lequel l'appareil comprend une pluralité de modules de culture cellulaire agencés les uns à côté des autres.

3. Appareil de culture cellulaire selon l'une quelconque des revendications précédentes, dans lequel le module de culture cellulaire comprend en outre une ou plusieurs saillies (280) fixées à et faisant saillie depuis la paroi sur le côté apical (231, 531) ou le côté basal (233, 533) de la paroi, ou fixées au et faisant saillie depuis le côté supérieur (232, 532) de la paroi inférieure de la deuxième chambre.

4. Appareil de culture cellulaire selon l'une quelconque des revendications précédentes, dans lequel la deuxième chambre comprend en outre un ou plusieurs moyens de culture de matériel biologique.

5. Appareil de culture cellulaire selon l'une quelconque des revendications précédentes, dans lequel la paroi comprend un ou plusieurs pores traversants (240) formés dans celle-ci et la paroi est agencée entre la première chambre et la deuxième chambre de sorte que la première chambre et la deuxième chambre soient en communication d'écoulement l'une avec l'autre par l'intermédiaire de l'un ou des plusieurs pores traversants.

6. Appareil de culture cellulaire selon l'une quelconque des revendications 1 à 5, dans lequel la partie inférieure de la deuxième chambre est fabriquée dans le matériau de partie inférieure élastique configuré pour se déformer dans la troisième direction, la troisième direction étant opposée à la deuxième direction, en réponse à une force appliquée sur le côté inférieur de la paroi inférieure de la deuxième chambre ; la première chambre comprend au moins deux compartiments et au moins une paroi de compartiment (260), dans lequel les au moins deux compartiments comprennent un premier compartiment (251) et un deuxième compartiment (252), dans lequel l'au moins une paroi de compartiment est agencée entre le premier compartiment et le deuxième compartiment de sorte que le premier compartiment et le deuxième compartiment soient en communication d'écoulement l'un avec l'autre ; et la paroi (214) comprend un ou plusieurs pores traversants formés dans celle-ci et la paroi (214) est agencée entre la première chambre et la deuxième chambre de sorte que la première chambre et la deuxième chambre soient en communication d'écoulement l'une avec l'autre par l'intermédiaire de l'un ou des plusieurs pores traversants.

7. Appareil de culture cellulaire selon l'une quelconque des revendications 1 à 6, dans lequel le matériau de paroi de la paroi est un matériau de paroi élastique configuré pour se déformer dans la deuxième direction en réponse à une force appliquée sur le côté première chambre de la paroi.

8. Appareil de culture cellulaire selon l'une quelconque des revendications 1 à 7, dans lequel la paroi inférieure de la deuxième chambre est fabriquée dans un matériau de paroi inférieure élastique configuré pour se déformer dans une troisième direction, la troisième direction étant opposée à la deuxième direction, en réponse à une force appliquée sur le côté inférieur de la paroi inférieure de la deuxième chambre.

9. Appareil de culture cellulaire selon l'une quelconque des revendications 1 à 8, dans lequel la deuxième chambre d'au moins un module de culture cellulaire de l'un ou des plusieurs modules de culture cellulaire comprend au moins une couche d'amélioration d'adhésion cellulaire fabriquée dans un matériau hydrophile, l'au moins une couche d'amélioration d'adhésion cellulaire est prévue sur une surface intérieure de la deuxième chambre, à condition que, si la paroi comprend un ou plusieurs pores traversants formés dans celle-ci, l'au moins une couche d'amélioration d'adhésion cellulaire soit placée sur la surface intérieure de la deuxième chambre de sorte que l'un ou les plusieurs pores traversants de la paroi restent ouverts.

10. Appareil de culture cellulaire selon l'une quelconque des revendications 1 à 9, dans lequel la paroi d'au moins un module de culture cellulaire de l'un ou des plusieurs modules de culture cellulaire comprend au moins une couche d'amélioration d'adhésion cellulaire fabriquée dans un matériau hydrophile, l'au moins une couche d'amélioration d'adhésion cellulaire étant prévue sur la paroi, à condition que, si la paroi comprend un ou plusieurs pores traversants formés dans celle-ci, l'au moins une couche d'amélioration d'adhésion cellulaire est prévue sur la paroi de sorte que l'un ou les plusieurs pores traversants de la paroi restent ouverts, dans lequel le matériau hydrophile de l'au moins une couche d'amélioration d'adhésion cellulaire est choisi sur la base d'au moins un type de cellules à cultiver sur la paroi.

11. Appareil de culture cellulaire selon l'une quelconque des revendications 1 à 10, dans lequel la première chambre dans au moins un module de culture cellulaire de l'un ou des plusieurs modules de culture cellulaire est mise en œuvre sous la forme d'un tube creux sans fond.

12. Appareil de culture cellulaire selon l'une quelconque des revendications 1 à 11, dans lequel la première chambre dans au moins un module de culture cellulaire de l'un ou des plusieurs modules de culture cellulaire est mise en œuvre sous la forme d'un tube creux présentant un fond incurvé ou incliné, le fond incurvé ou incliné présentant au moins une première cavité de chambre (524) et au moins un premier trou de chambre formé dans chacune de l'au moins une première cavité de chambre, et dans lequel la paroi inférieure de la deuxième chambre (508) est fabriquée dans un matériau de paroi inférieure élastique configuré pour se déformer dans une troisième direction, la troisième direction étant opposée à la deuxième direction, en réponse à une force appliquée sur le côté inférieur de la paroi inférieure de la deuxième chambre.

13. Appareil de culture cellulaire selon la revendication 12, dans lequel le fond incurvé ou incliné est recouvert, de l'extérieur, d'une couche d'amélioration d'adhésion cellulaire, de sorte que l'au moins un premier trou de chambre de l'au moins une première cavité de chambre reste ouvert, la couche d'amélioration d'adhésion cellulaire étant fabriquée dans un matériau hydrophile.

14. Appareil de culture cellulaire selon l'une quelconque des revendications 1 à 13, dans lequel la deuxième chambre d'au moins un module de culture cellulaire du un ou des plusieurs modules de culture cellulaire présente une hauteur se trouvant dans une plage comprise entre 50 µm et 350 µm.

15. Appareil de culture cellulaire selon l'une quelconque des revendications 1 à 14, dans lequel chacun du au moins deux réservoirs de milieu de culture dans au moins un module de culture cellulaire du un ou des modules de culture cellulaire est mis en œuvre sous la forme d'un tube creux.

16. Appareil de culture cellulaire selon l'une quelconque des revendications 1 à 15, dans lequel au moins un module de culture cellulaire du un ou des plusieurs modules de culture cellulaire comprend en outre au moins deux électrodes, dans lequel chacune des au moins deux électrodes est agencée indépendamment dans la première chambre, la deuxième chambre, dans un ou plusieurs des au moins deux réservoirs de milieu de culture, ou la paroi, ou une quelconque combinaison de ceux-ci.

17. Appareil de culture cellulaire selon l'une quelconque des revendications 1 à 16, dans lequel le un ou les plusieurs pores traversants de la paroi présentent une taille de pore moyenne se trouvant dans une plage comprise entre 0,2 µm et 200 µm.

18. Incubateur de culture cellulaire comprenant :
un appareil de culture cellulaire (100) tel que défini dans l'une quelconque des revendications 1 à 17 ; et
une station de pipetage (702) configurée pour alimenter en milieu de culture chacun des au moins deux réservoirs de milieu de culture dans chaque module de culture cellulaire du un ou des plusieurs modules de culture cellulaire.

19. Procédé pour affecter une culture cellulaire, le procédé comprenant :
(a) la fourniture d'un appareil de culture cellulaire tel que défini dans l'une quelconque des revendications 1 à 17, dans lequel chaque deuxième chambre de chaque module de culture cellulaire du un ou des plusieurs modules de culture cellulaire comprend une culture cellulaire comprenant une pluralité de cellules d'un premier type de cellules ;
(b) l'application d'une ou de plusieurs premières forces
i) sur un ou plusieurs premiers emplacements sur le côté inférieur de la paroi inférieure de la deuxième chambre, de sorte que la paroi inférieure de la deuxième chambre se déforme dans la troisième direction ; et/ou
ii) sur un ou plusieurs deuxièmes emplacements sur le côté première chambre de la paroi, de sorte que la paroi se déforme dans la deuxième direction,
éliminant ainsi au moins une cellule de la pluralité de cellules du premier type de cellules de la culture cellulaire, et formant une première culture cellulaire affectée.

20. Procédé selon la revendication 19, dans lequel le procédé comprend en outre les étapes consistant à :
(c) fournir un deuxième milieu de culture cellulaire comprenant un deuxième type de cellules à l'un des au moins deux réservoirs de milieu de chaque module de culture cellulaire du un ou des plusieurs modules de culture cellulaire ;
(d) faire en sorte que le deuxième milieu de culture s'écoule depuis ledit un des au moins deux réservoirs de milieu de culture par l'intermédiaire de la deuxième chambre vers le reste des au moins deux réservoirs de milieu de culture dans chaque module de culture cellulaire du un ou des plusieurs modules de culture cellulaire pendant une deuxième période de temps d'écoulement prédéfinie, la deuxième période de temps d'écoulement prédéfinie étant choisie sur la base du deuxième milieu de culture ; et
(e) incuber l'appareil de culture cellulaire pendant une deuxième période de temps d'incubation prédéfinie, formant ainsi une première culture cellulaire affectée cultivée, la deuxième période de temps d'incubation prédéfinie étant choisie sur la base du premier et du deuxième type de cellules.

21. Procédé selon la revendication 20, dans lequel, en (d), l'écoulement du deuxième milieu de culture cellulaire est provoqué par une unité d'entraînement d'écoulement.

22. Procédé selon l'une quelconque des revendications 19 à 21, dans lequel le deuxième type de cellules est identique ou différent du premier type de cellules et, en (e), la deuxième période de temps d'incubation prédéfinie est choisie sur la base du premier et du deuxième type de cellules.

23. Procédé selon l'une quelconque des revendications 19 à 22, dans lequel le procédé comprend en outre les étapes consistant à :
- appliquer une ou plusieurs deuxièmes forces
i) sur un ou plusieurs troisièmes emplacements sur le côté inférieur de la paroi inférieure de la deuxième chambre, le un ou les plusieurs troisièmes emplacements étant différents du un ou des premiers emplacements, de sorte que la paroi inférieure de la deuxième chambre se déforme dans la troisième direction ; et/ou
ii) sur un ou plusieurs quatrièmes emplacements sur le côté première chambre de la paroi, le un ou les quatrièmes emplacements étant différents du un ou des deuxièmes emplacements, de sorte que la paroi se déforme dans la deuxième direction,
éliminant ainsi au moins une cellule de la première culture cellulaire affectée ou de la première culture cellulaire affectée cultivée, et formant une deuxième culture cellulaire affectée ou une deuxième culture cellulaire affectée cultivée ;
- fournir un troisième milieu de culture cellulaire comprenant un troisième type de cellules à l'un des au moins deux réservoirs de milieu de chaque module de culture cellulaire du un ou des plusieurs modules de culture cellulaire ;
- faire en sorte que le troisième milieu de culture s'écoule depuis ledit un des au moins deux réservoirs de milieu de culture par l'intermédiaire de la deuxième chambre vers le reste des au moins deux réservoirs de milieu de culture dans chaque module de culture cellulaire du un ou des plusieurs modules de culture cellulaire pendant une troisième période de temps d'écoulement prédéfinie, la troisième période de temps d'écoulement prédéfinie étant choisie sur la base du troisième milieu de culture ; et
- incuber l'appareil de culture cellulaire pendant une troisième période de temps d'incubation prédéfinie, la troisième période de temps d'incubation prédéfinie étant choisie sur la base du premier type de cellules et/ou du troisième type de cellules.

24. Procédé selon l'une quelconque des revendications 19 à 23, dans lequel le procédé comprend en outre :
- la fourniture d'un quatrième milieu de culture cellulaire comprenant un quatrième type de cellules par l'intermédiaire de la première chambre de chaque module de culture cellulaire de l'un ou des plusieurs modules de culture cellulaire vers le côté première chambre de la paroi ; et
- l'incubation de l'appareil de culture cellulaire pendant une quatrième période de temps d'incubation prédéfinie, la quatrième période de temps d'incubation prédéfinie étant choisie sur la base du quatrième type de cellules,
dans lequel la paroi de chaque module de culture cellulaire de l'un ou des plusieurs modules de culture cellulaire comprend un ou plusieurs pores traversants formés dans celle-ci et la paroi est agencée entre la première chambre et la deuxième chambre de sorte que la première chambre et la deuxième chambre soient en communication d'écoulement l'une avec l'autre par l'intermédiaire de l'un ou des plusieurs pores traversants, et dans lequel une quelconque de l'application d'une ou de plusieurs force(s) est effectuée sur le un ou les plusieurs premiers et/ou troisièmes emplacements sur le côté inférieur de la paroi inférieure de la deuxième chambre.

25. Procédé selon l'une quelconque des revendications 19 à 24, dans lequel le procédé comprend en outre les étapes consistant à :
- fournir un quatrième milieu de culture cellulaire comprenant un quatrième type de cellules par l'intermédiaire d'un premier compartiment de la première chambre de chaque module de culture cellulaire de l'un ou des plusieurs modules de culture cellulaire vers le côté première chambre de la paroi ;
- fournir un cinquième milieu de culture cellulaire comprenant un cinquième type de cellules par l'intermédiaire d'un deuxième compartiment de la première chambre de chaque module de culture cellulaire de l'un ou des plusieurs modules de culture cellulaire sur le côté première chambre de la paroi ; et
- incuber l'appareil de culture cellulaire pendant une cinquième période de temps d'incubation prédéfinie, la cinquième période de temps d'incubation prédéfinie étant sélectionnée sur la base du quatrième et du cinquième type de cellules,
dans lequel la paroi de chaque module de culture cellulaire de l'un ou des plusieurs modules de culture cellulaire comprend un ou plusieurs pores traversants formés dans celle-ci et la paroi est agencée entre la première chambre et la deuxième chambre de sorte que la première chambre et la deuxième chambre soient en communication d'écoulement l'une avec l'autre par l'intermédiaire de l'un ou des plusieurs pores traversants, dans lequel la première chambre comprend l'au moins une paroi de compartiment, et dans lequel l'au moins une paroi est agencée entre le premier compartiment et le deuxième compartiment de sorte que le premier compartiment et le deuxième compartiment soient en communication d'écoulement l'un avec l'autre, et dans lequel une quelconque de l'application d'une ou de plusieurs force(s) est effectuée sur le un ou les plusieurs premiers et/ou troisièmes emplacements sur le côté inférieur de la paroi inférieure de la deuxième chambre.

26. Procédé selon l'une quelconque des revendications 19 à 25, dans lequel le premier, le deuxième, le troisième, le quatrième et/ou le cinquième type de cellules est choisi indépendamment dans le groupe consistant en astrocytes, organoïdes cérébraux, sphéroïdes cérébraux, cellules neuronales, cellules endothéliales, cellules épithéliales, cellules musculo-squelettiques, cellules souches pluripotentes induites (IPSC) dérivées ou différenciées, et cellules musculaires.
